(19) Europäisches Patentamt / European Patent Office / Office européen des brevets

(11) **EP 3 401 828 B1**

(12) **EUROPEAN PATENT SPECIFICATION**

(45) Date of publication and mention
of the grant of the patent:
**06.05.2020 Bulletin 2020/19**

(21) Application number: **16883711.0**

(22) Date of filing: **28.11.2016**

(51) Int Cl.:
*G06F 21/62* (2013.01)          *G09C 1/00* (2006.01)
*G06F 7/544* (2006.01)

(86) International application number:
**PCT/JP2016/085115**

(87) International publication number:
**WO 2017/119211 (13.07.2017 Gazette 2017/28)**

(54) **INFORMATION PROCESSING DEVICE, INFORMATION PROCESSING SYSTEM, INFORMATION PROCESSING METHOD AND PROGRAM**

INFORMATIONSVERARBEITUNGSVORRICHTUNG, INFORMATIONSVERARBEITUNGSSYSTEM, INFORMATIONSVERARBEITUNGSVERFAHREN UND PROGRAMM

DISPOSITIF DE TRAITEMENT D'INFORMATIONS, SYSTÈME DE TRAITEMENT D'INFORMATIONS, PROCÉDÉ ET PROGRAMME DE TRAITEMENT D'INFORMATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.01.2016 JP 2016001677**

(43) Date of publication of application:
**14.11.2018 Bulletin 2018/46**

(73) Proprietor: **Sony Corporation
Tokyo 108-0075 (JP)**

(72) Inventor: **KAWAMOTO, Yohei
Tokyo 108-0075 (JP)**

(74) Representative: **D Young & Co LLP
120 Holborn
London EC1N 2DY (GB)**

(56) References cited:
**JP-A- 2013 122 707     JP-A- 2014 206 696
JP-A- 2015 194 959**

- **ROB HALL ET AL: "Achieving Both Valid and Secure Logistic Regression Analysis on Aggregated Data from Different Private Sources", ARXIV.ORG, CORNELL UNIVERSITY LIBRARY, 201 OLIN LIBRARY CORNELL UNIVERSITY ITHACA, NY 14853, 30 November 2011 (2011-11-30), XP080553724,**

- **STEPHEN E FIENBERG ET AL: "Valid Statistical Analysis for Logistic Regression with Multiple Sources", 12 May 2008 (2008-05-12), PROTECTING PERSONS WHILE PROTECTING THE PEOPLE, SPRINGER BERLIN HEIDELBERG, BERLIN, HEIDELBERG, PAGE(S) 82 - 94, XP019134087, ISBN: 978-3-642-10232-5 * section 1, 2, 3.2 ***

- **DUVERLE DAVID A ET AL: "Privacy-Preserving Statistical Analysis by Exact Logistic Regression", 2015 IEEE SECURITY AND PRIVACY WORKSHOPS, IEEE, 21 May 2015 (2015-05-21), pages 7-16, XP033177673, DOI: 10.1109/SPW.2015.14 [retrieved on 2015-07-17]**

- **Cosma R Shalizi: "Logistic Regression and Newton's Method", Advanced Data Analysis, 15 March 2011 (2011-03-15), XP055524859, Retrieved from the Internet: URL:https://www.stat.cmu.edu/~cshalizi/402 /lectures/14-logistic-regression/lecture-1 4.pdf [retrieved on 2018-11-19]**

- **DOWSLEY RAFAEL ET AL: "A Two-Party Protocol with Trusted Initializer for Computing the Inner Product", 24 August 2010 (2010-08-24), INTERNATIONAL CONFERENCE ON SIMULATION, MODELING, AND PROGRAMMING FOR AUTONOMOUS ROBOTS,SIMPAR 2010; [LECTURE NOTES IN COMPUTER SCIENCE; LECT.NOTES COMPUTER], SPRINGER, BERLIN, HEIDELBERG, PAGE(S) 337 - 350, XP047428757, ISBN: 978-3-642-17318-9 * section 1 ***

**(Cont. next page)**

- GOLDREICH O ET AL: "How to play ANY mental game", PROCEEDINGS OF THE NINETEENTH ANNUAL ACM CONFERENCE ON THEORY OF COMPUTING , STOC '87, ACM PRESS, NEW YORK, NEW YORK, USA, 1 January 1987 (1987-01-01), pages 218-229, XP058087422, DOI: 10.1145/28395.28420 ISBN: 978-0-89791-221-1

- SHUANG WU ET AL.: 'Privacy-preserving Logistic Regression Analysis for Vertically Partitioned Data' 2014 NENDO PROCEEDINGS OF THE ANNUAL CONFERENCE OF JSAI (DAI 28 KAI 15 May 2014, pages 1 - 4, XP009507440

**Description**

TECHNICAL FIELD

The present disclosure relates to an information processing device, an information processing system, and an information processing method, and a program. More particularly, the present disclosure relates to an information processing device, an information processing system, and an information processing method that are capable of estimating, without disclosing a plurality of different pieces of secure data, the relationship between the pieces of secure data, and a program.

BACKGROUND ART

Logistic regression analysis has been known as a technique of predicting an outcome variable (y) from an explanatory variable (x).

Specifically, for example, the explanatory variable (x) is defined as a plurality of explanatory variables (x1 to x3):

(x1): gender of user (male = 1, female = 0),
(x2): age of user (from 0), and
(x3): cholesterol level of user (e.g., 150 to 250).

In addition, the outcome variable (y) is defined as one outcome variable (y1):
(y1): onset or non-onset of disease (e.g., hyperlipemia) (onset = 1, non-onset = 0).

An organization A (entity A), specifically, for example, the organization A (entity A) being an operator of a Web site can acquire the explanatory variables (x1 to x3) for a large number of users, for example, 100 people, on the basis of, for example, browsing information from browsing users of the Web site.

The explanatory variables corresponding to each user are personal information regarding each user, and thus are undesirable to release.

Meanwhile, a different organization B (entity B), for example, a hospital retains the outcome variable (y) for the one hundred users, namely, (y1): onset or non-onset of disease (e.g., hyperlipemia) (onset = 1, non-onset = 0).

The data retained in the hospital is also personal information, and thus should not be released.

Note that, data not to be released such as personal information is referred to as secure data or sensitive data.

The arrangement has difficulty in analyzing the relationship between the explanatory variable (x) and the outcome variable (y) because the different organizations retain the explanatory variable (x) and the outcome variable (y) individually.

However, for example, the outcome variable (y) is required to be estimated from arbitrary explanatory variables (x1 to x3) in some cases.

Specifically, for example, the operator of the Web site, being the organization A (entity A), outputs advertising for specific users, namely, "user targeted advertising" onto the Web site.

Specifically, performance of advertising output of providing a user estimated having (y1): onset of disease (e.g., hyperlipemia) with advertising for medicine for the disease (e.g., hyperlipemia) or preventive medicine can increase the possibility for purchase of the medicine, and thus more effective advertising output can be performed.

In this manner, in a case where the retainer of the explanatory variable (x) is different from the retainer of the outcome variable (y) and the two pieces of data are not allowed to be disclosed mutually, processing of estimating the outcome variable (y) more reliably from the explanatory variable (x) has high availability in variable fields.

The logistic regression analysis is one example of the estimation processing technique.

The retainer of the explanatory variable (x) is not allowed to receive the outcome variable (y) directly from the retainer of the outcome variable (y), but can perform analysis processing of estimating the outcome variable (y) more reliably from the explanatory variable (x) with reception of data including the outcome variable (y) subjected to cryptographic processing or conversion processing, namely, converted data (concealed data).

Examples of a conventional technology disclosing such analysis processing include Patent Document 1 (Japanese Patent Application Laid-Open No. 2011-83101) and Patent Document 2 (Japanese Patent Application Laid-Open No. 2009-199068).

Patent Document 1 (Japanese Patent Application Laid-Open No. 2011-83101) discloses a secret computation system that integrates a plurality of pieces of concealed data to perform statistical analysis.

Secret computation (secure computation) is used as a method of acquiring a statistic with the concealed data. However, there has not been provided a specific method of computing the statistic from the concealed data without mutual disclosure of information, and thus only a configuration relating to a framework for performing the secret computation, has been disclosed.

Concealment processing of data or secret computation (secure computation) processing with concealed data is intricate and increases in processing time in response to the volume of data, and thus there is a problem that processing cost is excessive.

In a case where a logistic regression parameter is estimated with the secret computation system disclosed in Patent Document 1, the estimation is considerably less efficient because typical secure computation remaining intact is used.

In addition, Patent Document 2 (Japanese Patent Application Laid-Open No. 2009-199068) discloses a secure computation (secure computation) system that calculates an arithmetic result f(m) of a logic circuit f(x) for an input value m, with the input value m remaining concealed, and discloses a specific logic circuit that performs secure computation. In a case where computation expressible with the logic circuit disclosed in Patent Document 2 is performed, the secure computation with the system disclosed in Patent Document 2 is available.

However, many different types of arithmetic processing, such as addition, subtraction, and multiplication, are required in order to estimate a logistic regression parameter, and thus there is a problem that expression of the arithmetic processing with a logic circuit, increases in circuit scale and increases in computational complexity.

In addition, there is a problem that typical secure computation that performs computation with an input value concealed, increases in computational complexity or in traffic, in response to the number of input values to be secret.

CITATION LIST

PATENT DOCUMENT

Patent Document 1: Japanese Patent Application Laid-Open No. 2011-83101
Patent Document 2: Japanese Patent Application Laid-Open No. 2009-199068

SUMMARY OF THE INVENTION

PROBLEMS TO BE SOLVED BY THE INVENTION

The present disclosure has been made in consideration of, for example, the problems, and an object of the present disclosure is to provide an information processing device, an information processing system, and an information processing method that are capable of efficiently performing, without disclosing a plurality of different pieces of secure data (concealed data), estimation of the relationship between the pieces of secure data, and a program.

Furthermore, an object of one embodiment of the present disclosure is to provide an information processing device, an information processing system, and an information processing method that efficiently perform estimation of a logistic regression parameter, and a program.

SOLUTIONS TO PROBLEMS

The invention is defined by the independent claims.

Note that, the program according to the present disclosure is provided to, for example, an information processing device or a computer system capable of executing various program codes, through a storage medium, for example. Execution of the program by a program execution unit on the information processing device or the computer system allows processing corresponding to the program to be achieved.

The features, the advantages, and another different object according to the present disclosure will be clear with the embodiment to be described later according to the present invention and the more detailed descriptions based on the attached drawings. Note that, a system in the present specification is a logical aggregate configuration including a plurality of devices, but is not limited to a configuration including the constituent devices in the same housing.

EFFECTS OF THE INVENTION

According to the configuration of one embodiment of the present disclosure, high-speed and efficient parameter calculation processing of a logistic regression model is achieved.

Specifically, a logistic regression parameter is calculated, the logistic regression parameter being a parameter of the logistic regression model indicating the relationship between an explanatory variable and an outcome variable being secure data corresponding to each sample. A data processing unit calculates the inner product ($t\_s$) of the explanatory variable and the outcome variable with application of secure computation being computation processing applied with converted data of each of the variables, and performs computation processing excluding the calculation processing of the inner product, as computation processing without the converted data, to calculate the logistic regression parameter in accordance with the maximum likelihood method with the Newton-Raphson method (iterative convergence method).

According to the present configuration, the high-speed and efficient parameter calculation processing of the logistic regression model is achieved.

Note that the effects described in the present specification are, but are not limited to, just exemplifications, and thus additional effects may be provided.

BRIEF DESCRIPTION OF DRAWINGS

Fig. 1 is a table for describing exemplary data for performing logistic regression analysis.

Fig. 2 is a diagram of an exemplary configuration of one information processing system that performs logistic regression analysis processing.

Fig. 3 is a diagram for describing exemplary respective pieces of data retained by information processing devices.

Fig. 4 is a diagram for describing learning data to be applied to the logistic regression analysis and a logistic regression model.

Fig. 5 is a table for describing exemplary sample unit data and profile unit data.

Fig. 6 is a diagram for describing exemplary processing of calculating an added result of secure data with secure computation.

Fig. 7 is a diagram for describing exemplary processing of calculating a multiplied result of the secure data with the secure computation.

Fig. 8 is a diagram for describing processing of estimating a parameter $\beta$ in accordance with the maximum likelihood method with the Newton-Raphson method (iterative convergence method).

Fig. 9 is a diagram of the configurations of parameter-calculation execution units 111 and 121 included in information processing device A 110 being an outcome-variable retaining device and the information processing device B 120 being an explanatory-variable retaining device, respectively.

Fig. 10 is a flowchart for describing a processing sequence to be performed by the information processing device according to the present disclosure.

Fig. 11 is a diagram for describing the processing of estimating the parameter $\beta$ in accordance with the maximum likelihood method with the Newton-Raphson method (iterative convergence method).

Fig. 12 is a flowchart for describing a processing sequence of estimating the parameter $\beta$ in accordance with the maximum likelihood method with the Newton-Raphson method (iterative convergence method).

Fig. 13 is a flowchart for describing a processing sequence of estimating the parameter $\beta$ in accordance with the maximum likelihood method with the Newton-Raphson method (iterative convergence method) with the secure computation reduced.

Fig. 14 is a diagram of an exemplary hardware configuration of an information processing device.

MODE FOR CARRYING OUT THE INVENTION

An information processing device, an information processing system, and an information processing method, and a program according to the present disclosure will be described in detail below with reference to the drawings. The descriptions will be given in accordance with the following items.

1. Outline of Logistic Regression Analysis
2. Parameter Estimation Processing with Logistic Regression Analysis
3. Estimation Processing of Logistic Regression Parameter with Maximum Likelihood Method
4. Estimation Method of Logistic Regression Parameter with Secure Computation
5. Estimation Method of Logistic Regression Parameter with Secure Computation Reduced
6. Reduction Effect in Computational Complexity of Parameter Calculation Processing according to Present Disclosure
7. Exemplary Hardware Configuration of Information Processing Device
8. Summary of Configuration of Present Disclosure

[1. Outline of Logistic Regression Analysis]

First, an outline of logistic regression analysis will be described.

The logistic regression analysis has been known as a technique of predicting an outcome variable (y) from an explanatory variable (x).

Processing with the logistic regression analysis will be described.

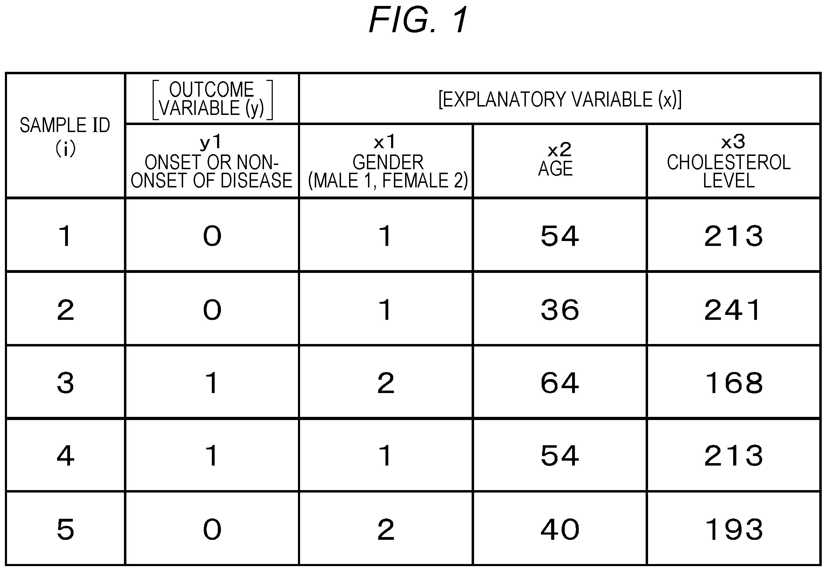

Fig. 1 illustrates exemplary data for performing the logistic regression analysis.

A list of an outcome variable (y) and an explanatory variable (x) for a plurality of samples (i) is illustrated. A sample i corresponds to, for example, one user i.

The outcome variable (y) includes onset or non-onset of disease, for example, hyperlipemia (onset = 1, non-onset = 0).

The explanatory variable (x) includes gender (x1), age (x2), and cholesterol level (x3).

As described above, an organization A (entity A), specifically, for example, the operator of a Web site can acquire the explanatory variables (x1 to x3) for a large number of users (samples (i)), for example, 100 people (i = 1 to 100), on the basis of, for example, browsing information from browsing users of the Web site.

The data generated and acquired by the organization A (entity A) on the basis of, for example, the browsing information from the browsing users of the Web site, is valuable in marketing. However, the data is information including personal information, and thus is undesirable to release. That is, the data is secure data (also referred to as, for example, sensitive data) and thus is to be prevented from leaking out.

Meanwhile, a different organization B (entity B), for example, a hospital retains the outcome variable (y) for the one hundred users (samples), namely, (y1): onset or non-onset of disease (e.g., hyperlipemia) (onset = 1, non-onset = 0).

The data retained by the hospital is also secure data, and thus is to be prevented from leaking out.

That is, the explanatory variables (x1 to x3) and the outcome variable (y1) illustrated in Fig. 1 are individually held by the different organizations, and each piece of data is the secure data to be prevented from leaking out.

Therefore, there is provided an arrangement in which a third party is not allowed to check the explanatory variables (x1 to x3) and the outcome variable (y1) together, similarly to the organizations A and B.

In such an arrangement, for example, the retainer of the explanatory variable (x) uses the logistic regression analysis in order to predict the outcome variable (y) from the explanatory variable (x).

Exemplary specific logistic regression analysis processing will be described.

As illustrated in Fig. 1, the explanatory variable (x) is defined as the plurality of explanatory variables (x1 to x3):

(x1): gender of user (male = 1, female = 0),

(x2): age of user (from 0), and

(x3): cholesterol level of user (e.g., 150 to 250). In addition, the outcome variable (y) is defined as the one outcome variable (y1):

(y1): onset or non-onset of disease (e.g., hyperlipemia) (onset = 1, non-onset = 0).

As described above, the organization A (entity A), specifically, for example, the operator of the Web site can acquire the explanatory variables (x1 to x3) for a large number of users, for example, 100 people, on the basis of, for example, the browsing information from the browsing users of the Web site.

However, the outcome variable (y) for the one hundred users, namely, (y1): onset or non-onset of disease (e.g., hyperlipemia) (onset = 1, non-onset = 0), is the secure data retained by the different organization B (entity B), for example, the hospital.

Therefore, the organization A (entity A) is not allowed to acquire the outcome variable (y) for the one hundred users.

Similarly, the retainer of the explanatory variable (x) being the secure data is not allowed to receive the outcome variable (y) from the retainer of the outcome variable (y) being the secure data. However, the retainer of the explanatory variable (x) is allowed to receive data including the outcome variable (y) subjected to cryptographic processing or conversion processing, namely, converted data (concealed data) of the secure data.

The retainer of the explanatory variable (x) receives the converted data (concealed data) of the outcome variable (y) and then performs various types of arithmetic, so that the outcome variable (y) associated with a predetermined explanatory variable (x) can be estimated.

One representative technique of the estimation processing is the logistic regression analysis.

The logistic regression analysis is one type of statistical regression model often used in medical science or social science, and is a data analysis technique for predicting an outcome variable from an explanatory variable.

In the logistic regression analysis, an expression of calculating the probability p(x) of occurrence of an event is set under a condition including observation values of the explanatory variable (x), such as (x1 to x3) illustrated in Fig. 1 given, and then a parameter in the set expression is calculated (estimated).

In the example illustrated in Fig. 1, the probability p(x) corresponds to the probability that the outcome variable (y1) is 1 indicating onset of disease, indicated as the outcome variable (y). That is, the probability p(x) indicates the probability of onset of disease. The probability p(x) has a value of 0 to 1.

Under a condition including the observation values (x1 to xr) of the explanatory variable (x) given, an expression of calculating the probability p(x) of occurrence of an event, is given in (Expression 1) below.

[Math. 1]

$$\text{logit } p(x) = \beta_0 + \beta_1 x_1 + \beta_2 x_2 + \cdots + \beta_r x_r$$

$$\ldots \ (\text{Expression 1})$$

Note that,

$$\text{logit } p(x) = \log\left(\frac{p(x)}{1 - p(x)}\right)$$

(Expression 1) above is referred to as a logistic regression model.

$x\_1, ..., x\_r$ represent explanatory variables in (Expression 1) above.

$\beta\_0, ..., \beta\_r$ represent logistic regression parameters. Hereinafter, the logistic regression parameters are simply referred to as parameters.

Note that, a character subsequent to an underscore (e.g., _0) represents a subscript in the following descriptions.

$\beta\_0, ..., \beta\_r$ represent $\beta_0$ to $\beta_r$, respectively.

Processing of estimating the parameters $\beta\_0, ..., \beta\_r$ in (Expression 1) above, is performed in the logistic regression analysis.

Determination of the parameters $\beta\_0, ..., \beta\_r$ enables the probability p(x) of occurrence of the event, to be calculated under the condition including the observation values ($x\_1, ..., x\_r$) of the explanatory variable (x) given, in accordance with (Expression 1) above.

[2. Parameter Estimation Processing with Logistic Regression Analysis]

Next, the parameter estimation processing with the logistic regression analysis will be described.

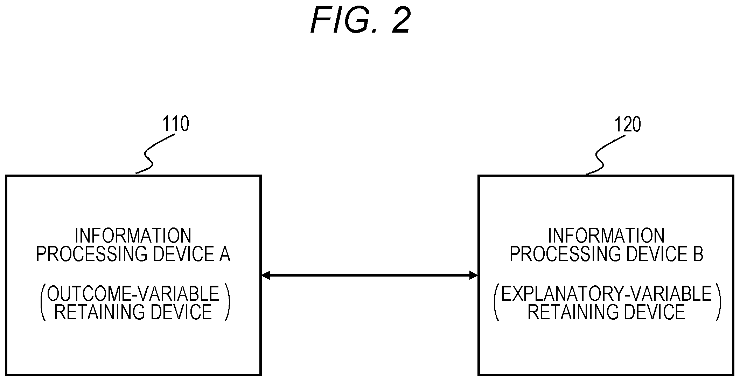

Fig. 2 is a diagram of an exemplary configuration of one information processing system that performs logistic regression analysis processing according to the present technology.

As illustrated in Fig. 2, two information processing devices A 110 and 120 are present.

The information processing device A 110 and the information processing device B 120 each retain only either the explanatory variable (x) or the outcome variable (y).

According to the present embodiment, the information processing device A 110 is an outcome-variable retaining device that retains the outcome variable (y) and the information processing device B 120 is an explanatory-variable retaining device that retains the explanatory variable (x).

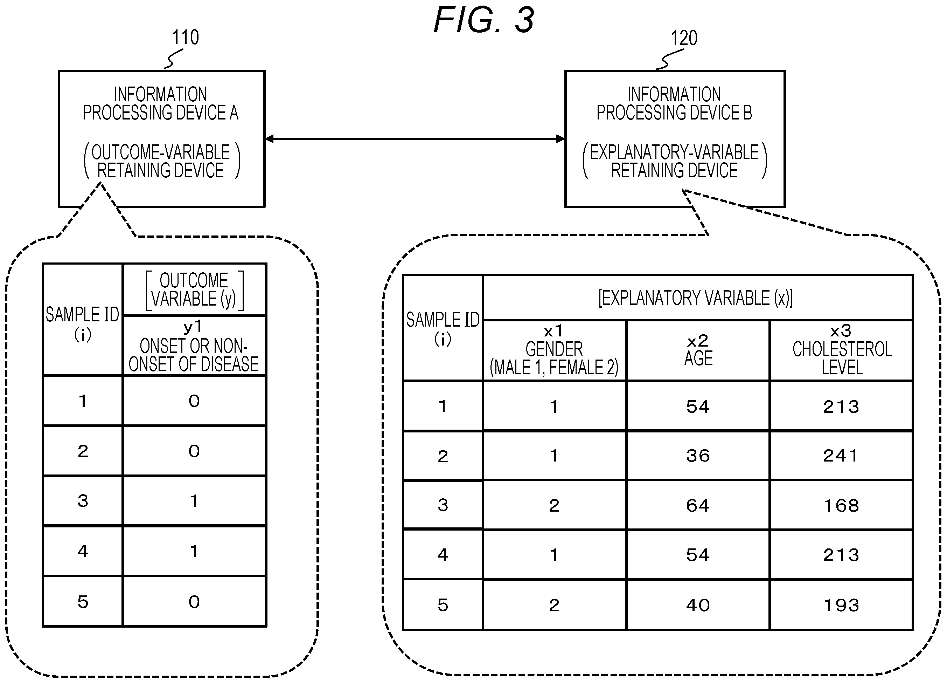

For example, the two information processing devices A 110 and 120 hold pieces of data as in Fig. 3. In a case where the pieces of data are personal data or sensitive data, the pieces of data are undesirable to release, from the viewpoint of protection of individual privacy.

In addition, the companies each are in a state where the data is an asset having an economic value and is undesirable to supply to a different company.

Meanwhile, there is a need for acquisition of much more knowledge with a data combination between different companies than individual use. In the processing to be described below according to the present disclosure, the two entities (information processing device A 110 and information processing device B 120) securely estimate the logistic regression parameters, namely, the parameters: $\beta\_0, ..., \beta\_r$ in (Expression 1) described earlier, without sharing the data itself mutually.

The processing to be described below according to the present technology enables the two entities (information processing device A 110 and information processing device B 120) to estimate the logistic regression parameters $\beta\_0$, ..., $\beta\_r$ without the mutual data sharing. The parameter estimation enables each of the entities (information processing device A 110 and information processing device B 120) to derive (estimate) the relationship between the explanatory variable (x) and the outcome variable (y).

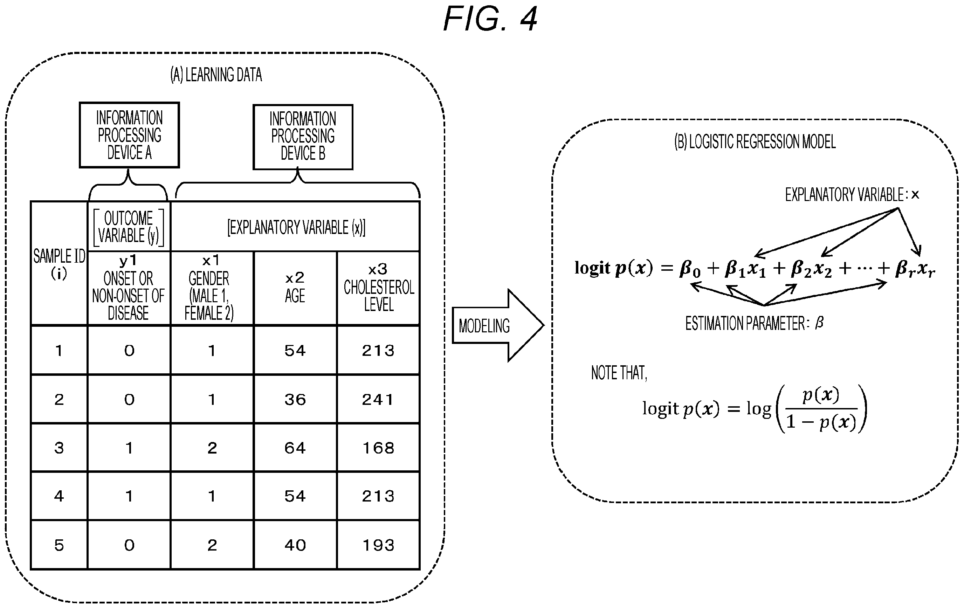

As illustrated in Fig. 4, in a case where the entities (information processing device A 110 and information processing device B 120) retain the explanatory variable (x) and the outcome variable (y) individually as secret data (secure data) for (A) learning data, application of (B) the logistic regression model enables, when a predetermined explanatory variable (x) is given, the outcome variable (y) for an element i (e.g., user i) given the explanatory variable (x), to be estimated, so that useful knowledge can be acquired.

Note that, the logistic regression model is the expression of calculating the event occurrence probability p(x) from the explanatory variable (x) and the logistic regression parameters $\beta\_0$, ..., $\beta\_r$, expressed in (Expression 1) described earlier. The event occurrence probability p(x) corresponds to, for example, the estimate (0 to 1) of the outcome variable (y).

Specifically, p(x) = 1 represents the outcome variable y = 1, namely, onset of disease, and p(x) = 0 represents the outcome variable y = 0, namely, non-onset of disease.

Estimation of the parameters $\beta\_0$, ..., $\beta\_r$ by the parameter estimation with the logistic regression model expressed in (Expression 1), setting of the estimated parameters into (Expression 1), and substitution of the explanatory variables (x1 to x3) of a user i (sample i) having the outcome variable (y) not acquired enable a value of 0 to 1 to be calculated for the event occurrence probability p(x).

If the calculated value p(x) is approximate to 1, a high possibility of onset of disease can be determined for the user i (sample i).

Meanwhile, if the calculated value p(x) is approximate to 0, a low possibility of onset of disease can be determined for the user i (sample i).

A specific embodiment for estimating the logistic regression parameters $\beta\_0$, ..., $\beta\_r$, will be described below.

Before the specific description, definition of terms and fundamental algorithms will be first described.

(2-1. Explanatory Variable)

(2-1-1) Parameter Estimation Algorithm for Explanatory Variable (x) being Continuous Variable

A continuous variable is a measurable variable in number or quantity, and is, for example, age, cholesterol level, or the like in the example illustrated in Fig. 1.

In this manner, in a case where the explanatory variable (x) is the continuous variable, the value of the explanatory variable (x) being the continuous variable, remaining intact may be substituted for the explanatory variables (x_1, ..., x_r) of the probability estimation expression based on (Expression 1) described earlier.

That is, for example, age data (54) indicating age, data (213) indicating cholesterol level, and the like in the explanatory variable (x) remaining intact may be substituted for the explanatory variables (x_1, ..., x_r) in (Expression 1).

(2-1-2) Parameter Estimation Algorithm for Explanatory Variable (x) being Categorical Variable

A categorical variable is an unmeasurable variable in number or quantity, and is, for example, data of gender or the like (e.g., male = 1, female = 0). In a case where two values to be taken by the categorical variable are provided, the value of the explanatory variable (x) is 0 or 1.

In this case, the value (0 or 1) of the explanatory variable (x) remaining intact may be substituted for the explanatory variables (x_1, ..., x_r) of the probability estimation expression based on (Expression 1) described earlier.

In a case where three or more values to be taken by the categorical variable are provided, for example, in a case where the explanatory variable (x) having three or more categories, such as residence (Tokyo, Kanagawa, Saitama, and the like), is used, the value of the explanatory variable (x) remaining intact cannot be substituted for the explanatory variables (x_1, ..., x_r) of the probability estimation expression based on (Expression 1) described earlier.

A category number of three or more in the j-th explanatory variable (x_j) is defined as K, and a categorical identifier is defined as k = 1, 2, ..., K.

At this time, K number of explanatory variables (x_jk) corresponding to the category number K, are set for the j-th explanatory variable (x_j), and the K number of explanatory variables (x_jk) in value are set as follows:

x_jk = 1: belonging to the k category of the j-th explanatory variable, and
x_jk = 0: not belonging to the k category of the j-th explanatory variable.

k includes 1 to K, and the explanatory variables (x_jk) are set in the same number as the category number K.

Furthermore, for the parameter $\beta$, parameters are set in corresponding number to the category number K in the j-th explanatory variable (x_j). That is, the parameter $\beta\_{jk}$ (k = 1, ..., K_j) is a parameter corresponding to the explanatory variable (x_jk).

The processing alters (Expression 1) described earlier, namely, the expression of calculating the probability p(x) of occurrence of the event under the condition including the observation values (x1 to xr) of the explanatory variable (x) given, into (Expression 2) below.

[Math. 2]

$$\text{logit}\, p(\boldsymbol{x}) = \beta_0 + \sum_{k=1}^{K_1} \beta_{1k} x_{1k} + \cdots + \sum_{k=1}^{K_r} \beta_{rk} x_{rk}$$

... (Expression 2)

Note that,

$$\text{logit}\, p(\boldsymbol{x}) = \log\left(\frac{p(\boldsymbol{x})}{1 - p(\boldsymbol{x})}\right)$$

In (Expression 2) above, $x\_1k$, ..., $x\_rk$ each are the explanatory variable of the category k (k = 1 to $K\_j$) of the event j (j = 1 to r).

The explanatory variable ($x\_jk$) is a provisional explanatory variable corresponding to the category, generated from the original explanatory variable ($x\_j$), and is also referred to as a dummy variable.

In addition, $\beta\_0$, $\beta\_1k$, ..., $\beta\_rk$ are logistic regression parameters.

Note that, $\beta\_1k$, ..., $\beta\_rk$ each are the logistic regression parameter corresponding to the explanatory variable of the category k (k = 1 to $K\_j$) of the event j (j = 1 to r).

Note that, for use of (Expression 2) above, the estimate of the parameter ($\beta\_jk$) corresponding to each category is ineffective for an absolute value, but is effective for a relative difference, and thus a first category parameter is typically set to zero, for example. Thus, the degree of freedom is K - 1 for the category number K.

(2-1-3) Parameter Estimation Algorithm for Explanatory Variable (x) Including Continuous Variable and Categorical Variable Mixed

Next, a parameter estimation algorithm for the explanatory variable (x) including the continuous variable and the categorical variable mixed, will be described.

Parameters to be set corresponding to the explanatory variable ($x\_j$) corresponding to the continuous variable and the explanatory variable ($x\_jk$) corresponding to the categorical variable, are as follows:

(a) a parameter ($\beta\_j$) corresponding to the explanatory variable ($x\_j$) corresponding to the continuous variable, and
(b) a parameter ($\beta\_jk$) corresponding to the explanatory variable ($x\_jk$) corresponding to the categorical variable.

The degree of freedom of each parameter (number of parameters to be estimated independently) is as follows:

(a) 1 for the parameter ($\beta\_j$) corresponding to the explanatory variable ($x\_j$) corresponding to the continuous variable, and
(b) K - 1 (category number = K) for each j for the parameter ($\beta\_jk$) corresponding to the explanatory variable ($x\_jk$) corresponding to the categorical variable.

Therefore, in a case where s number of explanatory variables ($x\_j$) corresponding to the continuous variable and t number of explanatory variables ($x\_jk$) corresponding to the categorical variable are mixed, the number of independent parameters relating to the s number of explanatory variables ($x\_j$) corresponding to the continuous variable is s in number and the number of independent parameters relating to the t number of explanatory variables ($x\_jk$) corresponding to the categorical variable with a category number of ($K\_j$) is ($K\_1$ - 1) + ($K\_2$ - 1) + ... + ($K\_t$ - 1) in number.

(2-1-4) Sample and Profile

Next, a sample being data to be used for the parameter estimation and a profile being an intermediate data structure to be generated from the sample, will be described.

The sample includes, for example, the samples (i) of Fig. 1, and includes, for example, the individual users.

Each of the samples (i) has j number of explanatory variables ($x\_j$) and at least one outcome variable (y) set in value.

(i) Sample

With the sample being n in size (number), the value of the outcome variable ($y\_i$) corresponding to the i-th sample (i = 1, ..., n), is defined as follows:

$y\_i$ = 1: occurrence of an event, and
$y\_i$ = 0: non-occurrence of the event.

Similarly, r number of explanatory variables ($x^i\_1$, $x^i\_2$, ..., $x^i\_r$) are ready for the explanatory variable ($x\_j$) corresponding to the i-th sample (i = 1, ..., n).

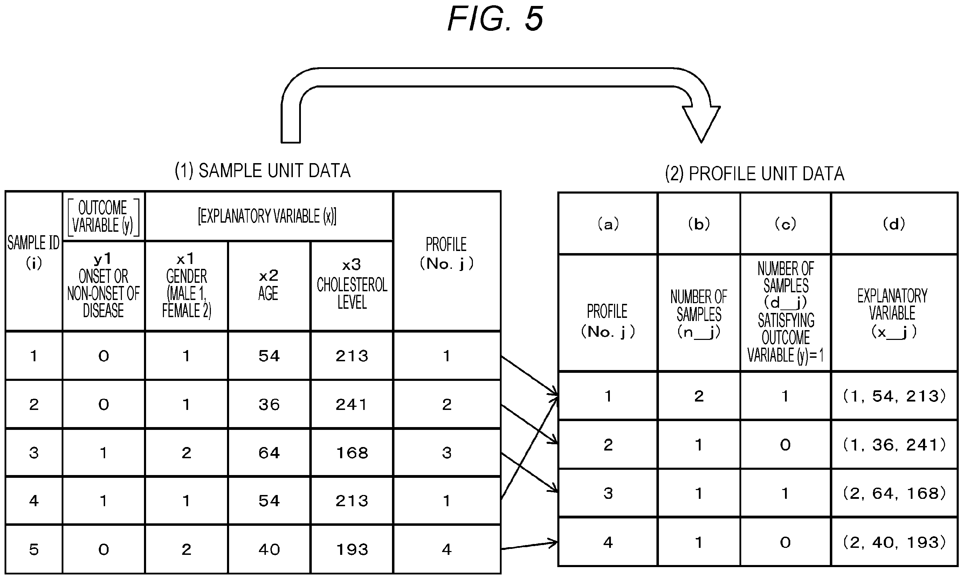

For example, the data is similar to (1) sample unit data illustrated on the left of Fig. 5.

The number of times of occurrence of the event corresponding to the number of samples satisfying that the value of the outcome variable (y) is 1, namely, satisfying $y\_i$ = 1, is expressed in (Expression 3) below.

[Math. 3]

$$f = \sum_{i=1}^{n} y_i \qquad \ldots \text{(Expression 3)}$$

(ii) Profile

A vector including the configuration values of the explanatory variables ($x^i\_1$, $x^i\_2$, ..., $x^1\_r$), note that i = 1 to n, is defined as an explanatory variable vector $x^i$.

For $x\_j$ (j = 1, ..., J), different patterns extracted and numbered from n number of explanatory variable vectors $x^i$ are referred to as the profile.

The profile extraction generates (2) profile unit data illustrated on the right of Fig. 5.

When the number of samples and the number of times of occurrence of the event in the profile $x\_j$ are defined as $n\_j$ and $d\_j$, respectively, (Expression 4) below is satisfied.

[Math. 4]

$$\sum_{j=1}^{J} n_j = n, \quad \sum_{j=1}^{J} d_j = f \qquad \ldots \text{(Expression 4)}$$

In (Expression 4) above, J represents the number of patterns of the explanatory variable occurring in the sample.

In addition, the following expression is defined: $x\_j$ = ($x\_j1$, ..., $x\_jr$).

(d), in (2) the profile unit data, includes data corresponding to the number of samples having the outcome variable (y) satisfying y = 1.

[3. Estimation Processing of Logistic Regression Parameter with Maximum Likelihood Method]

As described earlier, the estimation of the logistic regression parameters ($\beta\_0$, ..., $\beta\_r$) with (Expression 1) above, namely, (Expression 1) based on the logistic regression model, enables, when values of the explanatory variable (x) are given, the outcome variable (y) corresponding to the explanatory variable more reliably.

(Expression 1: the logistic regression model) above is the expression of calculating the probability p(x) of occurrence of the event with arithmetic of the observation values (x1 to xr) of the explanatory variable (x) and the logistic regression parameters ($\beta\_0$, ..., $\beta\_r$).

A method of estimating the parameter $\beta$ = $\beta\_0$, ..., $\beta\_r$ with the maximum likelihood method in a case where the sample and the profile have been given, will be first described.

For example, the method is parameter estimation processing in a case where all the data illustrated in Fig. 1 or Fig. 4(A) has been grasped.

That is, for example, the method of estimating, in a case where one organization (entity) retains data including both an outcome variable value and an explanatory variable value and a storage unit in an information processing device available to the one organization (entity) stores data including the outcome variable value and the explanatory variable value for a plurality of samples, the parameter $\beta = \beta\_0, ..., \beta\_r$ with the maximum likelihood method with the data will be described.

The likelihood of a group having the profile x_j observed, is defined in (Expression 5) below.

[Math. 5]

$$p(x_j)^{d_j}\left(1 - p(x_j)\right)^{n_j - d_j}$$

... (Expression 5)

With the likelihood of the group having the profile x_j observed is defined in (Expression 5) above, the entire likelihood is expressed in (Expression 6) below.

[Math. 6]

$$\text{like}(\boldsymbol{\beta}) = \prod_{j=1}^{J} p(x_j)^{d_j}\left(1 - p(x_j)\right)^{n_j - d_j}$$

... (Expression 6)

The maximum likelihood method finds the most suitable value of the parameter $\beta$ when the samples are given. That is, the value of the parameter $\beta$ at which the likelihood of the observed data set is maximum is found from all available values of the parameter $\beta$.

Specifically, a maximum likelihood estimate $\beta\_ML$ maximizing a likelihood function like($\beta$) is acquired to estimate the parameter $\beta$ maximizing the likelihood. (Expression 7) below is used for the computation.

[Math. 7]

$$L(\boldsymbol{\beta}) = \log\{\text{like}(\boldsymbol{\beta})\} = \sum_{j=1}^{J}\left\{d_j\log\left(p(x_j)\right) + (n_j - d_j)\log\left(1 - p(x_j)\right)\right\}$$

$$= \sum_{j=1}^{J}\left\{d_j(1, x^t)\boldsymbol{\beta} + n_j\log\left(1 - p(x_j)\right)\right\}$$

... (Expression 7)

Simultaneous equations in which (Expression 7) above differentiated partially with respect to the parameter $\beta$ is defined as zero, are only required to be solved.

That is, simultaneous equations in (Expression 8) below are solved.

[Math. 8]

$$\frac{dL}{d\beta_0} = \sum_{j=1}^{J}\left(d_j - n_j p(x_j)\right) = 0$$

$$\frac{dL}{d\beta_s} = \sum_{j=1}^{J} x_{js}\left(d_j - n_j p(x_j)\right) = 0 \qquad s = 1, ..., r.$$

... (Expression 8)

Because the simultaneous equations expressed in (Expression 8) above are nonlinear with respect to the parameter $\beta$, $\beta$ is acquired by linear approximation of Taylor expansion with the Newton-Raphson method (iterative convergence method).

The parameter $\beta$ is calculated with the Newton-Raphson method (iterative convergence method). Typically, the solution of the maximum likelihood estimate of the parameter $\beta$ can be calculated by iterative computation below.

[Math. 9]

$$\beta^{(k+1)} = \beta^{(k)} + I^{-1}\big(\beta^{(k)}\big)S\big(\beta^{(k)}\big)$$

... (Expression 9)

(Expression 9) above is repeated until (Expression 10) below is satisfied.

Note that, k in (Expression 9) above represents the number of repetitions.

An appropriate arbitrary value is set to a parameter initial value: $\beta^{(k)}$ with k = 0, and then the iterative computation starts.

[Math. 10]

$$\left|\{L(\beta^{(k+1)}) - L(\beta^{(k)})\}/L(\beta^{(k)})\right| < \varepsilon \quad (= \text{approximately } 0.00001)$$

... (Expression 10)

The iterative computation of (Expression 9) above until the satisfaction of (Expression 10) above, can acquire the parameter β.

The meaning of each variable is expressed in (Expression 11) below.

[Math. 11]

$$\Sigma(\beta) = I^{-1}(\beta) = (X^t V X)^{-1}$$

$$S(\beta) = \left(\frac{dL}{d\beta_0}, \frac{dL}{d\beta_1}, \dots, \frac{dL}{d\beta_r}\right)$$

$$X = \begin{bmatrix} 1 & x_{11} & \cdots & x_{1r} \\ 1 & x_{21} & \cdots & x_{2r} \\ \vdots & & \vdots & \\ 1 & x_{J1} & \cdots & x_{Jr} \end{bmatrix}$$

$$V = \begin{bmatrix} n_1 \hat{p}(x_1)(1 - \hat{p}(x_1)) & 0 & \cdots & 0 \\ 0 & n_2 \hat{p}(x_2)(1 - \hat{p}(x_2)) & \vdots & \vdots \\ \vdots & 0 & \vdots & \vdots \\ \vdots & \vdots & \vdots & \vdots \\ \vdots & \vdots & \vdots & 0 \\ 0 & 0 & \cdots & n_J \hat{p}(x_J)(1 - \hat{p}(x_J)) \end{bmatrix}$$

... (Expression 11)

The technique described above is a parameter estimation method in the situation in which the explanatory variable (x) and the outcome variable (y) both are known.

However, as described above, practically, the explanatory variable (x) and the outcome variable (y) each are often the secure data, such as personal data, and thus the situation in which the explanatory variable (x) and the outcome variable (y) both are known is often difficult to acquire.

A parameter estimation method in that case will be described below.

[4. Estimation Method of Logistic Regression Parameter with Secure Computation]

Next, a method of estimating the parameter β = β_0, ..., β_r with the maximum likelihood method with secure computation, in a case where the pieces of data of the explanatory variable (x) and the outcome variable (y) are separately retained by, for example, different organizations and the pieces of data are not allowed to be disclosed mutually as illustrated in Fig. 3, will be described.

As described earlier with reference to Fig. 3, in a case where the pieces of data of the explanatory variable (x) and the outcome variable (y) are personal data or sensitive data, the pieces of data are undesirable to release, from the viewpoint of protection of individual privacy. That is, the pieces of data are the secure data.

In addition, the companies each are in a state where the data is an asset having an economic value and is undesirable to supply to a different company.

Meanwhile, there is a need for acquisition of much more knowledge with a data combination between different companies than individual use.

Processing will be described below in which the two entities (information processing device A 110 and information processing device B 120) illustrated in Fig. 3 securely estimate the logistic regression parameters, namely, the parameters: $\beta\_0, .., \beta\_r$ in (Expression 1) described earlier, without mutually sharing the secure data including the explanatory variable (x) and the outcome variable (y).

The processing to be described below is that the two entities (information processing device A 110 and information processing device B 120) estimate the logistic regression parameters $\beta\_0, ..., \beta\_r$ without the mutually sharing of the secure data.

The parameter estimation enables each of the entities (information processing device A 110 and information processing device B 120) to derive (estimate) the relationship between the explanatory variable (x) and the outcome variable (y).

The two different devices each retaining only either the explanatory variable (x) or the outcome variable (y) performs data conversion, such as encryption, to its own explanatory variable (x) or outcome variable (y), to provide the other device with converted data.

The logistic regression parameters $\beta\_0, ..., \beta\_r$ set in the logistic regression model, namely, (Expression 1) described above are estimated with application of the converted data.

In this manner, without performing the sharing processing of the secure data, such as the explanatory variable (x) or the outcome variable (y), each of the entities (information processing device A 110 and information processing device B 120) performs arithmetic processing with the converted data of the secure data to acquire various arithmetic results of the secure data, such as an added result, a multiplied result, and an inner product of the secure data, for example.

Note that, the computation processing with the converted data of the secure data is referred to as the secure computation.

For the secure computation, the converted data of the secure data is used instead of the secure data itself. Various types of converted data, such as encrypted data and segmented data of the secure data, for example, are provided as the converted data.

An example of the secure computation is a GMW scheme described in Non-Patent Document 1 (O. Goldreich, S. Micali, and A. Wigderson. How to play any mental game. STOC'87, pp.218-229, 1987.), for example.

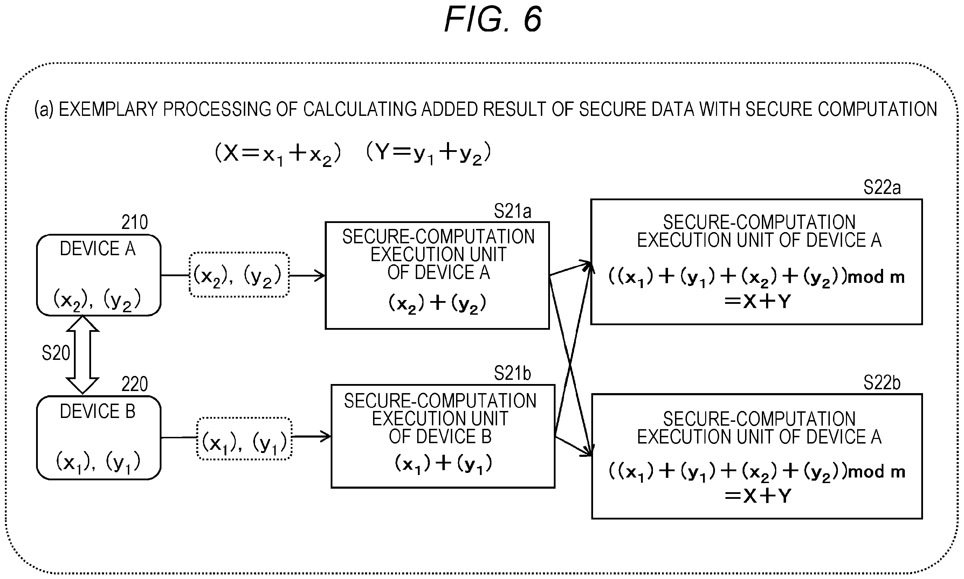

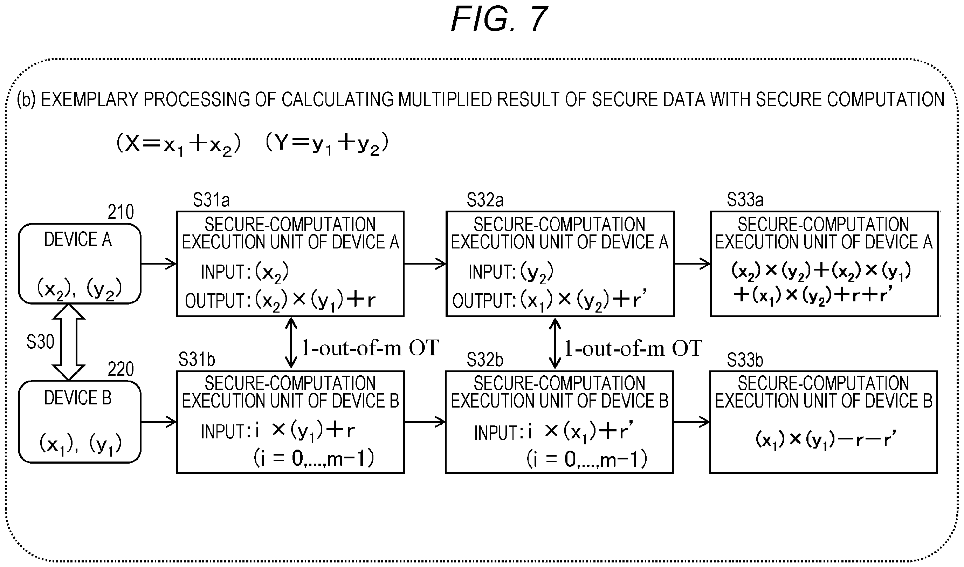

An outline of secure computation processing based on the GMW scheme will be described with reference to Figs. 6 and 7.

Fig. 6 is a diagram of exemplary processing of calculating an added value of the secure data with the secure computation based on the GMW scheme.

A device A 210 retains secure data X (e.g., explanatory variable (x)).

In addition, a device B 220 retains secure data Y (e.g., outcome variable (y)).

The secure data X and the secure data Y are the secure data, such as personal data, undesirable to release.

The device A 210 segments the secure data X into two pieces of data as below. Note that X is set as residual data of a predetermined numerical value m: mod m. $X = ((x\_1) + (x\_2)) \bmod m$

In the above expression, $(x\_1)$ is selected from 0 to (m - 1) uniformly and randomly and $(x\_2)$ is determined to satisfy the following expression: $(x\_2) = (X - (x\_1)) \bmod m$.

In this manner, the two pieces of segmented data $(x\_1)$ and $(x\_2)$ are generated.

Note that, here, the data to be segmented is, for example, the value (1) of gender of a sample (user) in the secure data illustrated in Fig. 1, and various different modes of segmented data can be set, for example, segmentation of the value (1) into (30) and (71) or into (45) and (56) for m = 100.

The value (0) of gender can be subjected to processing such as segmentation into (40) and (60) as a segmented value.

Age (54) can be subjected to processing such as segmentation into (10) and (44) or can be subjected to other various types of segmentation processing.

An important thing is that the original secure data (explanatory variable) is prevented from being specified from individual converted data (here, one piece of segmented data).

For example, the segmented data is not released as a set, and, for example, only one piece of segmented data is released, namely, is provided to the other device.

Meanwhile, the device B 220 also segments the secure data Y into two pieces of data as below:

$$Y = ((y\_1) + (y\_2)) \mod\_m.$$

In the above expression, (y_1) is selected from 0 to (m - 1) uniformly and randomly, and (y_2) is determined to satisfy the following expression: (y_2) = (Y - (y_1))mod m.

In this manner, the two pieces of segmented data (y_1) and (y_2) are generated.

As illustrated in Fig. 6, the device A 210 and the device B 220 each provide the other device with part of the segmented data, at step S20.

The device A 210 provides the device B 220 with the segmented data (x_1).

Meanwhile, the device B 220 provides the device A 210 with the segmented data (y_2).

X and Y each are the secure data, and thus are not allowed to leak.

However, even if only one piece of data of the pieces of segmented data (x_1) and (x_2) of X is acquired, the secure data X cannot be specified.

Similarly, even if only one piece of data of the pieces of segmented data (y_1) and (y_2) of Y is acquired, the secure data Y cannot be specified.

Therefore, only partial data of the segmented data of the secure data, is insufficient to specification of the secure data, and thus is allowed to be output outward.

In this manner, the device A 210 outputs the segmented data (x_1) to a computation-processing execution unit of the device B 220.

Meanwhile, the device B 220 outputs the segmented data (y_2) to a computation-processing execution unit of the device A 210.

(Step S21a)

At step S21a, the computation-processing execution unit of the device A 210 performs the following inter-segmented-data addition processing with the segmented data:

$$((x\_2) + (y\_2)) \mod m.$$

The device A 210 outputs an added result thereof to the computation-processing execution unit of the device B 220.

(Step S21b)

Meanwhile, at step S21b, the computation-processing execution unit of the device B 220 performs the following inter-segmented-data addition processing with the segmented data:

$$((x\_1) + (y\_1)) \mod m.$$

The device B 220 outputs an added result thereof to the computation-processing execution unit of the device A 210.

(Step S22a)

Next, at step S22a, the computation-processing execution unit of the device A 210 performs the following processing.

Two added results are further added, the two added results including: (1) the added result (x_2) + (y_2) of the segmented data calculated at step S21a; and (2) the added result (x_1) + (y_1) of the segmented data input from the device B 220. That is, the following computation is performed.

$$((x\_1)+(y\_1)+(x\_2)+(y\_2)) \mod m$$

The total added value of the segmented data is equivalent to the added value of the original secure data X and secure data Y.

That is, the following expression is satisfied:

$$((x\_1) + (y\_1) + (x\_2) + (y\_2)) \bmod m = X + Y.$$

(Step S22b)

Meanwhile, at step S22b, the computation-processing execution unit of the device B 220 performs the following processing.

Two added results are further added, the two added results including: (1) the added result (x_1) + (y_1) of the segmented data calculated at step S21b; and (2) the added result (x_2) + (y_2) of the segmented data input from the device A 210. That is, the following computation is performed.

$$((x\_1)+(y\_1)+(x\_2)+(y\_2)) \bmod m$$

The total added value of the segmented data is equivalent to the added value of the original secure data X and secure data Y.

That is, the following expression is satisfied:

$$((x\_1) + (y\_1) + (x\_2) + (y\_2)) \bmod m = X + Y.$$

In this manner, both the device A and the device B can calculate, without outputting the secure data X and the secure data Y outward, respectively, the added value of the secure data X and the secure data Y, namely, X + Y.

The processing illustrated in Fig. 6 is exemplary processing of calculating the added value of the secure data, applied with the secure computation based on the GMW scheme.

Note that, the processing described with reference to Fig. 6 includes an outline of the processing of calculating the added value of the secure data X and the secure data Yin a simple manner. For performance of practical addition processing or multiplication processing of the secure data, typically, the secure computation is required to be performed repeatedly, for example, application of a computed result acquired by first secure computation, to an input value of the next secure computation.

Fig. 7 is a diagram of exemplary processing of calculating a multiplied value of the secure data with the secure computation based on the GMW scheme.

The device A 210 retains the secure data X.

In addition, the device B 220 retains the secure data Y.

The secure data X and the secure data Y are the secure data undesirable to release.

The device A 210 segments the secure data X into two pieces of data:

$$X = ((x\_1) + (x\_2)) \bmod m.$$

In this manner, the secure data X is randomly segmented to generate the two pieces of segmented data (x_1) and (x_2).

Meanwhile, the device B 220 also segments the secure data Y into two pieces of data:

$$Y = ((y\_1) + (y\_2)) \bmod m.$$

In this manner, the secure data Y is randomly segmented to generate the two pieces of segmented data (y_1) and (y_2).

At step S30 illustrated in Fig. 7, the device A 210 provides the computation-processing execution unit of the device B 220 with the segmented data (x_1).

Meanwhile, the device B 220 provides the computation-processing execution unit of the device A 210 with the segmented data (y_2).

X and Y are the secure data, and thus are not allowed to leak.

However, even if only one piece of data of the pieces of segmented data (x_1) and (x_2) of X is acquired, the secure data X cannot be specified.

Similarly, even if only one piece of data of the pieces of segmented data (y_1) and (y_2) of Y is acquired, the secure data Y cannot be specified.

Therefore, only partial data of the segmented data of the secure data, is insufficient to specification of the secure data, and thus is allowed to be output outward.

In this manner, the device A 210 outputs the segmented data (x_1) to the computation-processing execution unit of the device B 220.

Meanwhile, the device B 220 outputs the segmented data (y_2) to the computation-processing execution unit of the device A 210.

Processing in the computation-processing execution unit of the device A 210 will be described.

The device A 210 retains the pieces of segmented data (x_1) and (x_2) of X and the segmented data (y_1) of Y received from the device B 220.

The processing is performed by the following procedure.

(Step S31a)

The computation-processing execution unit of the device A 210 performs [1-out-of-m OT] having an input/output value setting including an input value being x_2 and an output value M(x_2) satisfying $M_{(x\_2)} = (x\_2) \times (y\_1) + r$, together with the device B 220.

Note that, [1-out-of-m Oblivious Transfer (OT)] is an arithmetic protocol for performing the following processing.

Two entities being a sender and a selector are present.

The sender has an input value (M_0, M_1, ..., M_(m - 1)) including m number of elements.

The selector has an input value being $\sigma \in \{0, 1, ..., m - 1\}$.

The selector requests the sender having the m number of elements to send one element, so that the selector can acquire only the value of one element M_$\sigma$. The other (m - 1) number of elements: M_i ($i \neq \sigma$) are not allowed to be acquired.

Meanwhile, the sender is not allowed to know the input value $\sigma$ of the selector.

In this manner, the [1-out-of-m OT] protocol is intended for performing arithmetic processing with the transmission and reception of only one element from the m number of elements, and has a setting for preventing which one of the m number of elements has been transmitted and received, from being specified on the element reception side.

(Step S32a)

The computation-processing execution unit of the device A 210 performs [1-out-of-m OT] having an input/output value setting including an input value being y_2 and an output value M_(y_2)' satisfying $M_{(y\_2)}' = (x\_1) \times (y\_2) + r'$, together with the device B 220.

(Step S33a)

As the output value of the device A 210, an output value: M_(x_2) + M_(y_2) is computed in accordance with the following expression:

$$M\_(x\_2) + M\_(y\_2) = ((x\_2) \times (y\_2) + (x\_2) \times (y\_1) + r + (x\_1) \times (y\_2) + r') \bmod m.$$

Processing in the computation-processing execution unit of the other device B 220 will be described.

The device B 220 retains the pieces of segmented data (y_1) and (y_2) of Y and the segmented data (x_1) of X received from the device A 210.

The processing is performed by the following procedure.

(Step S31b)

With selection of a random number $r \in \{0, ..., m - 1\}$, an input value string to be used for [1-out-of-m OT] is generated on the basis of the segmented value y_1 of the secure data Y, the input value string being $i \times (y\_1) + r$, note that, i = 0, 1, ..., (m - 1).

Specifically, the following input value strings: M_0 to M_(m - 1) are generated:

$$M\_0 = 0 \times (y\_1) + r,$$

$$M\_1 = 1 \times (y\_1) + r,$$

$$\cdots,$$

and

$$M\_(m - 1) = (m - 1) \times (y\_1) + r.$$

The input value strings are generated.

Furthermore, the computation-processing execution unit of the device B 220 performs [1-out-of-m OT] based on the setting at step S31a described above, together with the device A 210.

(Step S32b)

With selection of a random number r' $\in$ {0, ..., m - 1}, an input value string to be used for [1-out-of-m OT] is generated on the basis of the segmented value y_1, the input value string being i × (x_1) + r', note that, i = 0, 1, ..., (m - 1) .

Specifically, the following input value strings: M'_0 to M'_(m - 1) are generated:

$$M'\_0 = 0 \times (x\_1) + r',$$

$$M'\_1 = 1 \times (x\_1) + r'$$

$$\cdots,$$

and

$$M'\_(m - 1) = (m - 1) \times (x\_1) + r'.$$

The input value strings are generated.

Furthermore, the computation-processing execution unit of the device B 220 performs [1-out-of-m OT] based on the setting at step S32a described above, together with the device A 210.

(Step S33b)

The following output value is calculated as the output value of the device B 220:

$$((x\_1) \times (y\_1) - r - r') \bmod m.$$

The value is calculated as the output value of the device B 220.

The following computation processing with the output value calculated by the device A 210 at step S33a and the output value calculated by the device B 220 at step S33b can calculate the multiplied value X × Y of the secure data X and the secure data Y:

$$(((x\_2) \times (y\_2) + (x\_2) \times (y\_1) + r + (x\_1) \times (y\_2)$$
$$+ r') + ((x\_1) \times (y\_1) - r - r')$$
$$= ((x\_1) + (x\_2)) \times ((y\_1) + (y\_2))$$
$$= X \times Y.$$

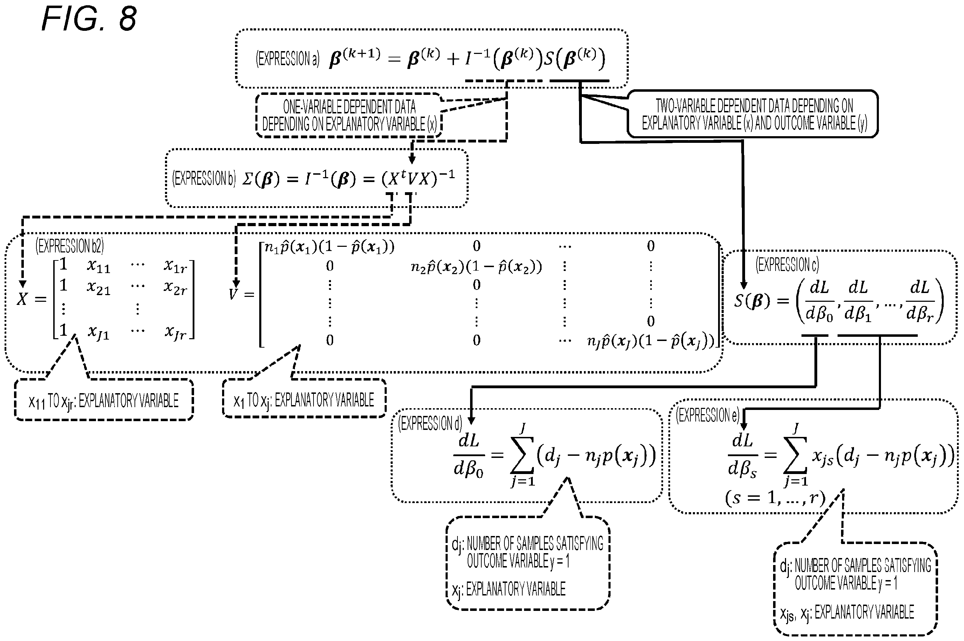

The mutual provision of the calculated result at step S33a and the calculated result at step S33b between the device A 210 and the device B 220 can calculate the multiplied value X × Y of the secure data X and the secure data Y.
In this manner, both the device A and the device B can calculate, without outputting the secure data X and the secure data Y outward, respectively, the multiplied value of the secure data X and the secure data Y, namely, XY.
The processing illustrated in Fig. 7 is exemplary processing of calculating the multiplied value of the secure data, applied with the secure computation based on the GMW scheme.
Note that, the processing described with reference to Fig. 7 includes an outline of the processing of calculating the multiplied value of the secure data X and the secure data Y in a simple manner. For practical addition processing or multiplication processing of the secure data, typically, the secure computation is required to be performed repeatedly, for example, by applying a computed result acquired by first secure computation, to an input value of the next secure computation.
In addition, the exemplary secure computation processing illustrated in Fig. 6 or 7 is an example of the secure computation, and other various different types of computation processing can be applied for modes of the secure computation.
Exemplary secure computation will be described with reference to Fig. 8 for the estimation of the parameter β = β_0, ..., β_r with the maximum likelihood method with the secure calculation in a case where the pieces of data of the explanatory variable (x) and the outcome variable (y) are separately retained by, for example, different organizations and the pieces of data are not allowed to be disclosed mutually as illustrated in Fig. 3 described earlier.

(Expression a) illustrated in Fig. 8 corresponds to (Expression 9) described earlier.

That is, (Expression a) is intended for estimating the parameter β in accordance with the maximum likelihood method with the Newton-Raphson method (iterative convergence method).
The parameter β is calculated with the Newton-Raphson method (iterative convergence method). Typically, the solution of the maximum likelihood estimate of the parameter β can be calculated by iterative computation of (Expression a) below.
[Math. 12]

$$\beta^{(k+1)} = \beta^{(k)} + I^{-1}\big(\beta^{(k)}\big)S\big(\beta^{(k)}\big) \qquad \text{... (Expression a)}$$

(Expression a) above is repeated until (Expression a2) below is satisfied.
[Math. 13]

$$\left|\{L(\beta^{(k+1)}) - L(\beta^{(k)})\}/L(\beta^{(k)})\right| < \varepsilon \ (= \text{approximately } 0.00001)$$

$$\text{... (Expression a2)}$$

The iterative computation of (Expression a) above until the satisfaction of (Expression a2) above, can acquire the parameter β.
(Expression a) above can be expanded as illustrated in Fig. 8.
As illustrated in Fig. 8, (Expression a) above includes (Expression b) and (Expression c) illustrated in Fig. 8, namely, the following expressions.
[Math. 14]

$$\Sigma(\beta) = I^{-1}(\beta) = (X^t V X)^{-1} \qquad \text{... (Expression b)}$$

$$S(\pmb{\beta}) = \left( \frac{dL}{d\beta_0}, \frac{dL}{d\beta_1}, \dots, \frac{dL}{d\beta_r} \right)$$

... (Expression c)

Furthermore, (Expression b) above includes matrices X and V expressed in (Expression b2) below.
[Math. 15]

$$X = \begin{bmatrix} 1 & x_{11} & \cdots & x_{1r} \\ 1 & x_{21} & \cdots & x_{2r} \\ \vdots & & \vdots & \\ 1 & x_{J1} & \cdots & x_{Jr} \end{bmatrix}$$

$$V = \begin{bmatrix} n_1\hat{p}(x_1)(1-\hat{p}(x_1)) & 0 & \cdots & 0 \\ 0 & n_2\hat{p}(x_2)(1-\hat{p}(x_2)) & \vdots & \vdots \\ \vdots & 0 & \vdots & \vdots \\ \vdots & \vdots & \vdots & \vdots \\ \vdots & \vdots & \vdots & 0 \\ 0 & 0 & \cdots & n_J\hat{p}(x_J)(1-\hat{p}(x_J)) \end{bmatrix}$$

As illustrated in Fig. 8, the matrices X and V expressed in (Expression b2) each include the explanatory variable (x) being the secure data as matrix elements or configuration data of matrix elements.
In addition, (Expression c) above includes (Expression d) and (Expression e) below as illustrated in Fig. 8.
[Math. 16]

$$\frac{dL}{d\beta_0} = \sum_{j=1}^{J} \left( d_j - n_j p(x_j) \right) = 0$$

... (Expression d)

$$\frac{dL}{d\beta_s} = \sum_{j=1}^{J} x_{js} \left( d_j - n_j p(x_j) \right) = 0 \qquad s = 1, \dots, r.$$

... (Expression e)

(Expression d) and (Expression e) above correspond to the simultaneous equations in (Expression 8) described earlier. That is, (Expression d) and (Expression e) correspond to the simultaneous equations in which L($\beta$) = log{like($\beta$)} = ... in (Expression 7) for acquiring the maximum likelihood estimate $\beta$_ML maximizing the likelihood function like($\beta$), differentiated partially with respect to $\beta$, is defined as 0.
As illustrated in Fig. 8, the simultaneous equations include the data (d) based on the outcome variable (y) being the secure data and the explanatory variable (x).
Note that, (d_j) included in (Expression d) and (Expression e) of Fig. 8 corresponds to (d) in (2) the profile unit data illustrated on the right of Fig. 5 described earlier with reference to Fig. 5, and includes the data corresponding to the number of samples having the outcome variable (y) satisfying y = 1.
As described above, the iterative computation of (Expression a) illustrated in Fig. 8 until the satisfaction of (Expression a2) above, acquires the parameter $\beta$ in the estimation processing of the logistic regression parameter.
However, as illustrated in Fig. 8, the explanatory variable (x) and the outcome variable (y) as the secure data are used in quantities in (Expression a).

The secure data, namely, the explanatory variable (x) and the outcome variable (y) individually retained by the two different information processing devices, are not allowed to be shared or released.

Therefore, without use of the explanatory variable (x) and the outcome variable (y) remaining intact, the iterative computation processing of (Expression a) illustrated in Fig. 8 until the satisfaction of (Expression a2) above, is required to be performed as arithmetic with the converted data generated from the explanatory variable (x) and the outcome variable (y), namely, the secure computation.

The secure computation performs computation applied with the converted data of each piece of secure data input or output between the devices, for example, generation of the converted data of the secure data (e.g., segmented data) and input or output of the converted data between the devices, as described with reference to Figs. 6 and 7.

For example, the matrix X and the matrix V expressed in Fig. 8 each include a large number of explanatory variables. Each of the explanatory variables is the secure data.

Therefore, in order to perform the secure computation, there is a need to generate the converted data, such as the segmented data, for each of the explanatory variables included in the matrix X and the matrix V illustrated in Fig. 8, input or output the converted data between the devices, and perform computation with the converted data.

For (Expression d) and (Expression e) illustrated in Fig. 8, similarly, there is a need to generate the converted data, such as the segmented data, individually for the explanatory variable (x) and the outcome variable (y) included as the constituent elements of the expressions, input or output the converted data between the devices, and perform computation with the converted data.

The throughput of such data conversion processing, data input/output processing, or furthermore computation processing with the converted data, increases as the amount of secure data to be applied to the secure computation increases.

Therefore, for a large amount of secure data, the iterative computation processing of (Expression a) illustrated in Fig. 8 needs a plenty of computational time and a plenty of computational resources. That is, there is a problem that the computational cost increases.

[5. Estimation Method of Logistic Regression Parameter with Secure Computation Reduced]

As described above, in a case where the pieces of data of the explanatory variable (x) and the outcome variable (y) are separately retained by, for example, the different organizations and the pieces of data are not allowed to be disclosed mutually, the estimation of the parameter $\beta = \beta\_0, ..., \beta\_r$ with the secure computation needs a plenty of computational time and a plenty of computational resources, and thus has a problem that the computational cost increases.

A configuration having a solution for the problem, namely, processing capable of estimating the logistic regression parameter $\beta = \beta\_0, ..., \beta\_r$ with reduction of the computational complexity of the secure computation without mutual disclosure of the pieces of data of the explanatory variable (x) and the outcome variable (y), will be described below.

As described earlier with reference to Fig. 3, in a case where the pieces of data of the explanatory variable (x) and the outcome variable (y) are personal data or sensitive data, the pieces of data are undesirable to release, from the viewpoint of protection of individual privacy.

In addition, the companies each are in a state where the data is an asset having an economic value and is undesirable to supply to a different company.

Meanwhile, there is a need for acquisition of much more knowledge with a data combination between different companies than individual use. In the processing to be described below according to the present disclosure, the two entities (information processing device A 110 and information processing device B 120) illustrated in Fig. 3 securely estimate the logistic regression parameters $\beta\_0, ..., \beta\_r$ with reduction of the computational complexity of the secure computation, without sharing the data itself mutually.

Note that, setting the estimated parameters into, for example, the logistic regression model (Expression 1 described above), enables the probability p(x) from various values of the explanatory variable (x), namely, the estimate of the outcome variable (y) to be calculated.

That is, each of the entities (information processing device A 110 and information processing device B 120) can estimate the relationship between the explanatory variable (x) and the outcome variable (y).

The two different devices each retaining only either the explanatory variable (x) or the outcome variable (y) performs data conversion, such as encryption, to its own explanatory variable (x) or outcome variable (y), to provide the other device with converted data.

The logistic regression parameters $\beta\_0, ..., \beta\_r$ set in the logistic regression model, namely, (Expression 1) described above are estimated with application of the converted data.

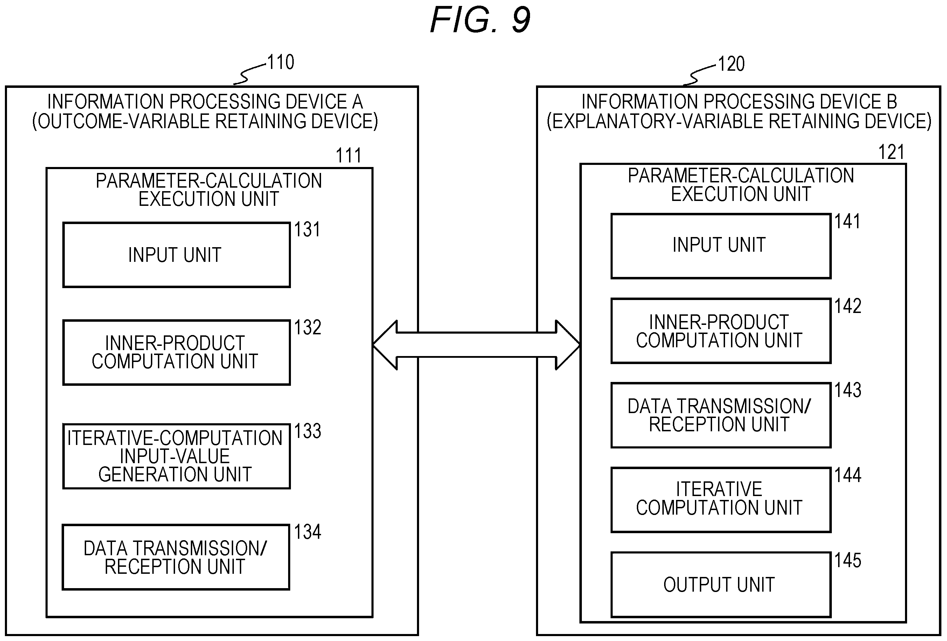

Fig. 9 illustrates a partial configuration of the information processing device A 110 being the outcome-variable retaining device and the information processing device B 120 being the explanatory-variable retaining device.

Fig. 9 illustrates parameter-calculation execution units 111 and 121 each being a data processing unit that performs the parameter estimation processing.

The parameter-calculation execution units 111 and 121 perform the parameter estimation without leaking the explanatory variable (x) and the outcome variable (y) outward.

The parameter-calculation execution unit 111 of the information processing device A 110 being the outcome-variable retaining device, includes an input unit 131, an inner-product computation unit 132, an iterative-computation input-value generation unit 133, and a data transmission/reception unit 134.

Meanwhile, the parameter-calculation execution unit 121 of the information processing device B 120 being the explanatory-variable retaining device, includes an input unit 141, an inner-product computation unit 142, a data transmission/reception unit 143, an iterative computation unit 144, and an output unit 145.

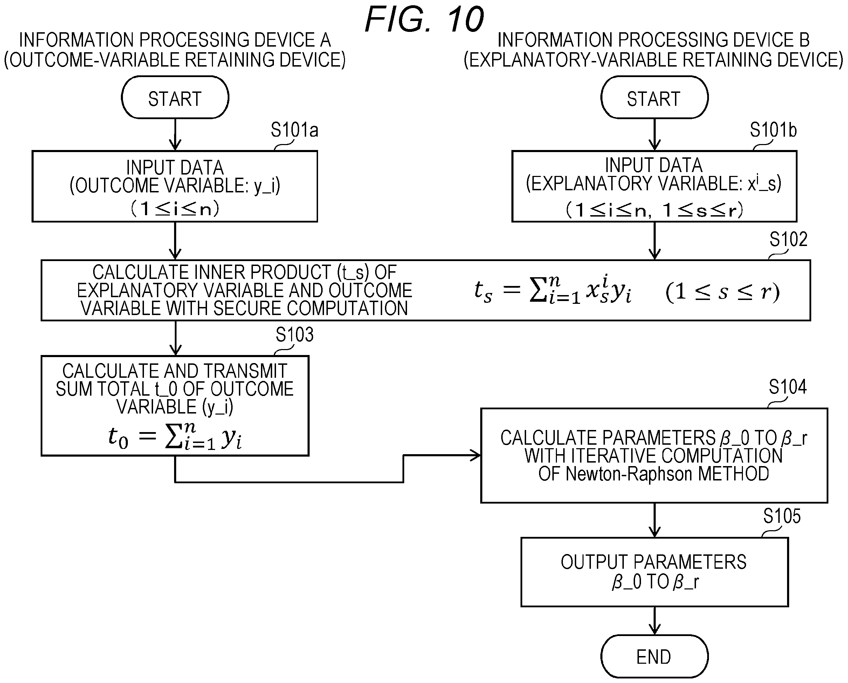

Fig. 10 is a flowchart for describing the sequence of the estimation processing of the logistic regression parameter $\beta = \beta\_0, ..., \beta\_r$ with the devices illustrated in Fig. 9.

That is, the flowchart describes the processing sequence of estimating the logistic regression parameter $\beta = \beta\_0, ..., \beta\_r$ in the logistic regression model (Expression 1), with the maximum likelihood method.

The sequence of the calculation processing of the logistic regression parameter $\beta = \beta\_0, ..., \beta\_r$ with the maximum likelihood method, will be specifically described below with reference to the block diagram illustrated in Fig. 9 and the flowchart illustrated in Fig. 10.

(a. Setting)

The element (i) and the explanatory variable (x) and the outcome variable (y) set corresponding to each element, included in the data to be subjected to the calculation processing of the logistic regression parameter $\beta = \beta\_0, ..., \beta\_r$ in the logistic regression model (Expression 1), are set as follows:

For n number of samples and the i-th sample (i = 1, ..., n),

outcome variable: $y\_i \in \{0, 1\}$ and

explanatory variable: r number of variables $(x^i\_1, x^i\_2, ..., x^i\_r)$ .

The explanatory variable and the outcome variable are associated with each other.

The information processing device A 110 retains data $y\_i$ (i = 1, ..., n) including an outcome variable value.

The information processing device B 120 retains data $(x^i\_1, x^i\_2, ..., x^i\_r)$ (i = 1, ..., n) including an explanatory variable value.

The pieces of data are the secure data not allowed to be released.

The logistic regression parameter $\beta = \beta\_0, ..., \beta\_r$ is estimated without mutual disclosure of the outcome variable and the explanatory variable individually retained by the devices.

(b. Procedure)

Next, the procedure of the estimation processing of the logistic regression parameter $\beta = \beta\_0, ..., \beta\_r$ will be described.

The processing at each step in the flowchart illustrated in Fig. 10, will be described sequentially.

(Step S101)

The processing at step S101 includes data input processing of the input units.

At step S101a, the input unit 131 of the parameter-calculation execution unit 111 in the information processing device A 110 being the outcome-variable (y) retaining device illustrated in Fig. 9 acquires the outcome variable $y\_i$ (note that, i = 1, ..., n) retained in a storage unit of the information processing device A 110, from the storage unit, to input the outcome variable $y\_i$ into the parameter-calculation execution unit 111.

Meanwhile, at step S101b, the input unit 141 of the parameter-calculation execution unit 121 in the information processing device B 120 being the explanatory variable (x) retaining device acquires the explanatory variables $(x^i\_1, x^i\_2, ..., x^i\_r)$ (note that, i = 1, ..., n) retained in a storage unit of the information processing device B 120, from the storage unit, to input the explanatory variables $(x^i\_1, x^i\_2, ..., x^i\_r)$ into the parameter-calculation execution unit 121.

(Step S102)

The processing at step S102 includes processing to be performed by the inner-product computation units 132 and 142 in the parameter-calculation execution units 111 and 121 of the information processing device A 110 and the information processing device B 120, respectively.

The inner-product computation units 132 and 142 calculate the inner product $(t\_s)$ of the explanatory variable (x) and the outcome variable (y), in accordance with (Expression 12) below.

[Math. 17]

$$t_s = \sum_{i=1}^{n} x_s^i y_i \quad (s = 1, \ldots, r)$$

... (Expression 12)

Note that, because the explanatory variable (x) and the outcome variable (y) both are the secure data subject to restriction of release, the calculation processing of the inner product (t_s) based on (Expression 12) above is performed with arithmetic not applied directly with the explanatory variable (x) and the outcome variable (y) being the secure data, namely, the secure computation applied with the converted data of the explanatory variable (x) and the outcome variable (y) as described with reference to Figs. 6 and 7.

The calculation processing of the inner product (t_s) based on (Expression 12) above, is performed with the secure computation not using directly the data y_i (i = 1, ..., n) including the outcome variable value, being the input value of the information processing device A 110, and the data ($x^i$_1, $x^i$_2, ..., $x^i$_r) (i = 1, ..., n) including the explanatory variable value, being the input value of the information processing device B 120.

As described earlier with reference to Figs. 6 and 7, the secure computation is the computation processing capable of acquiring various arithmetic results of the secure data, such as an added result, a multiplied result, or the inner product of the secure data, for example, with arithmetic with the converted data to be generated on the basis of the secure data, without direct use of the secure data not allowed to be released.

Note that, the inner product (t_s) of the explanatory variable (x) and the outcome variable (y) expressed in (Expression 12) above can be expressed in (Expression 13) below including (d) in (2) the profile unit data illustrated on the right of Fig. 5 described earlier with reference to Fig. 5, namely, the data (d) corresponding to the number of samples having the outcome variable (y) satisfying y = 1.

[Math. 18]

$$t_s = \sum_{i=1}^{n} x_s^i y_i = \sum_{j=1}^{J} x_{js} d_j$$

$$(s = 1, \ldots, r)$$

... (Expression 13)

The arithmetic applied with d expressed in (Expression 13) above, namely, the arithmetic expression applied with the data d corresponding to the number of samples having the outcome variable (y) satisfying y = 1, is included in part of (Expression e) in the computational expression for estimating the parameter $\beta$ in accordance with the maximum likelihood method with the Newton-Raphson method (iterative convergence method) described earlier with reference to Fig. 8.

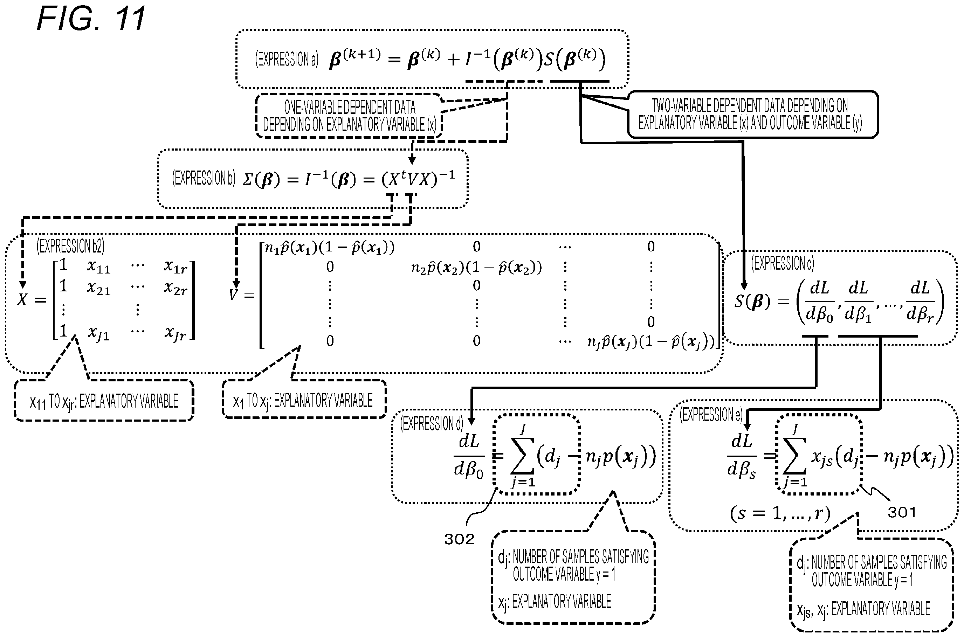

Fig. 11 illustrates a computation processing configuration for estimating the parameter $\beta$ in accordance with the maximum likelihood method with the same Newton-Raphson method as in Fig. 8 describe earlier.

As illustrated in Fig. 11, the arithmetic expression applied with the data d, for calculating the inner product (t_s) of the explanatory variable (x) and the outcome variable (y) in (Expression 13) above, corresponds to an arithmetic expression 301 in (Expression e) in Fig. 11.

The calculation processing of the inner product (t_s) to be performed at step S102, namely, the calculation processing of the inner product (t_s) of the explanatory variable (x) and the outcome variable (y) corresponds to processing of performing, as the secure computation, the arithmetic expression 301 in (Expression e) in Fig. 11.

Note that, as described above, for the secure computation, the converted data of the secure data is used instead of the secure data itself.

Various types of converted data, such as encrypted data of the secure data and the segmented data described with reference to Figs. 6 and 7, for example, are provided as the converted data.

Figs. 6 and 7 described earlier each illustrate exemplary secure computation processing based on the GMW scheme being one technique of the secure computation with the segmented data of the secure data.

Fig. 6 is the diagram of the exemplary processing of calculating the added value of the secure data with the secure computation based on the GMW scheme.

In addition, Fig. 7 is the diagram of the exemplary processing of calculating the multiplied value of the secure data with the secure computation based on the GMW scheme.

As described with reference to Figs. 6 and 7, the device A and the device B retaining different secure data not allowed to be disclosed, can calculate, without outputting the secure data X and the secure data Y outward, respectively, a mutual-secure-data arithmetic result, such as the added value or multiplied value of the secure data X and the secure data Y, with the secure computation.

The processing at step S102 illustrated in the flowchart of Fig. 10 includes the processing of calculating the inner product (t_s) of the explanatory variable (x) and the outcome variable (y) with the secure computation, to be performed by the inner-product computation units 132 and 142 in the parameter-calculation execution units 111 and 121 of the information processing device A 110 and the information processing device B 120. Specifically, the processing includes the processing of calculating the arithmetic expression expressed in (Expression 12) or (Expression 13), namely, the arithmetic expression 301 in (Expression e) in Fig. 11, with the secure computation.

A combination of the processing of calculating the added value of the secure data X and the secure data Y described earlier with reference to Fig. 6 and the processing of calculating the multiplied value of the secure data X and the secure data Y described with reference to Fig. 7 enables the inner product (t_s) of the explanatory variable (x) and the outcome variable (y) to be calculated.

That is, at step S102, the information processing device A 110 and the information processing device B 120 each output only the converted data to the other device to calculate the inner product (t_s) of the explanatory variable (x) and the outcome variable (y) with the secure computation, without mutual disclosure of the value of the outcome variable (y) and the value of the explanatory variable (x) being the secure data retained by the devices.

(Step S103)

Next, at step S103 of the flow illustrated in Fig. 10, the iterative-computation input-value generation unit 133 of the parameter-calculation execution unit 111 in the information processing device A 110 being the outcome-variable (y) retaining device calculates the sum total (t_0) of the outcome variable (y) in accordance with (Expression 14) below to output the calculated value to the parameter-calculation execution unit 121 in the information processing device B 120 through the data transmission/reception unit 134.

[Math. 19]

$$t_0 = \sum_{i=1}^{n} y_i$$

$$\ldots \text{(Expression 14)}$$

The data transmission/reception unit 143 of the parameter-calculation execution unit 121 in the information processing device B 120 being the explanatory-variable (x) retaining device receives the sum total (t_0) of the outcome variable (y) transmitted by the information processing device A.

Note that, the sum total (t_0) of the outcome variable (y) expressed in (Expression 14) above can be expressed in (Expression 15) below including (d) in (2) the profile unit data illustrated on the right of Fig. 5 described earlier with reference to Fig. 5, namely, the data (d) corresponding to the number of samples having the outcome variable (y) satisfying y = 1.

[Math. 20]

$$t_0 = \sum_{i=1}^{n} y_i = \sum_{j=1}^{J} d_j$$

$$\ldots \text{(Expression 15)}$$

The arithmetic applied with d expressed in (Expression 15) above, namely, the arithmetic expression applied with the data d corresponding to the number of samples having the outcome variable (y) satisfying y = 1, is included in part of (Expression d) expressed in the computational expression for estimating the parameter β in accordance with the maximum likelihood method with the Newton-Raphson method (iterative convergence method) described earlier with reference to Fig. 8.

As illustrated in Fig. 11 illustrating the Newton-Raphson method (iterative convergence method) similar to that of Fig. 8, the arithmetic expression applied with the data d, for calculating the sum total (t_0) of the outcome variable (y) in (Expression 15) above, corresponds to an arithmetic expression 302 in (Expression d) in Fig. 11.

The calculation processing of the sum total (t_0) of the outcome variable (y), to be performed at step S103, corresponds to processing of performing the arithmetic expression 302 in (Expression d) in Fig. 11.

Note that, because the processing at step S103 is performed inside the information processing device A 110 being the outcome-variable (y) retaining device, the processing is not required to be performed as the secure computation.

That is, without performance of generation processing of the converted data of the outcome variable (y) and output processing of the converted data to the external device, the processing at step S103 can be performed to calculate the sum total (t_0) of the outcome variable (y), in the arithmetic device inside the information processing device A 110 with acquisition of the outcome variable (y) being the secure data retained inside the information processing device A 110 and application of the acquired outcome variable (y) remaining intact.

Note that, the sum total (t_0) of the outcome variable (y) is not the secure data and thus can be output outward.

In this manner, the information processing device A 110 being the outcome-variable (y) retaining device calculates the sum total (t_0) of the outcome variable (y) with the typical arithmetic processing applied with the secure data, instead of the secure computation to output the sum total (t_0) of the outcome variable (y) to the information processing device B.

Such typical arithmetic processing can make a considerable reduction in computational time or computational resources in comparison to performance of the secure computation.

The iterative-computation input-value generation unit 133 in the information processing device A 110 calculates the sum total (t_0) of the outcome variable (y) in accordance with (Expression 14) or (Expression 15) described above to output the calculated value to the parameter-calculation execution unit 121 in the information processing device B 120 through the data transmission/reception unit 134.

(Step S104)

Next, at step S104, the iterative computation unit 144 of the parameter-calculation execution unit 121 in the information processing device B 120 being the explanatory-variable (x) retaining device performs the iterative computation of the Newton-Raphson method to the expression based on the logistic regression model expressed in (Expression 1) described earlier to perform updating and calculation processing of the logistic regression parameter $\beta\_1$ (i = 0, 1, ..., r).

Specifically, computation for (a) and (b) expressed in (Expression 17) below is repeated until (Expression 16) below is satisfied in terms of preset $\varepsilon$ (e.g., $\varepsilon$ = 0.00001) .

[Math. 21]

$$\left| \left\{ L(\boldsymbol{\beta}^{(k+1)}) - L(\boldsymbol{\beta}^{(k)}) \right\} / L(\boldsymbol{\beta}^{(k)}) \right| < \varepsilon \qquad \text{... (Expression 16)}$$

[Math. 22]

(a) calculate $S(\beta^{(k)})$ on the basis of $t_s$ ($0 \le s \le r$)

$$\frac{dL}{d\beta_0} = t_0 - \sum_{j=1}^{J} n_j p(\boldsymbol{x}_j)$$

$$\frac{dL}{d\beta_s} = t_s - \sum_{j=1}^{J} x_{js} n_j p(\boldsymbol{x}_j) \, (1 \le s \le r)$$

(b) calculate $\beta^{(k+1)}$

$$\boldsymbol{\beta}^{(k+1)} = \boldsymbol{\beta}^{(k)} + I^{-1}(\boldsymbol{\beta}^{(k)}) S(\boldsymbol{\beta}^{(k)}) \qquad \text{... (Expression 17)}$$

The repeating computation for (a) and (b) expressed in (Expression 17) until the satisfaction of (Expression 16) above updates the logistic regression parameter $\beta\_1$ (i = 0, 1, ..., r) and determines, as an output parameter, the parameter at the point in time when (Expression 16) above is satisfied.

Note that, an appropriate arbitrary value may be set to the parameter initial value: $\beta^{(0)}$ in (Expression 16) and (Expression 17) above.

In addition, the meaning of each symbol expressed in (Expression 16) and (Expression 17) above is the same as that of each symbol expressed in (Expression 6) to (Expression 11) described earlier as the estimation processing of the logistic regression parameter based on the maximum likelihood method. For example, the following expression is provided:

$$\mathtt{L(\beta) = log\{like(\beta)\}.}$$

At step S104, the processing to be performed by the iterative computation unit 144 of the parameter-calculation execution unit 121 in the information processing device B 120 being the explanatory-variable (x) retaining device includes the iterative computation of the Newton-Raphson method illustrated in Fig. 11, and is similar to the processing of Fig. 8 described earlier.

However, no secure computation is required in the iterative computation of the Newton-Raphson method at step S104.

Also at step S104, for example, the matrix X and the matrix V are computed in the iterative computation of the Newton-Raphson method illustrated in Fig. 11. The matrices each include the explanatory variable (x) being the secure data.

However, the information processing device B 120 being the explanatory-variable retaining device performs the processing at step S104.

The information processing device B 120 being the explanatory-variable retaining device sets the matrix X and the matrix V expressed in (Expression b2) of Fig. 11 with application of the explanatory variable (x) remaining intact, retained in the storage unit of the information processing device B 120, so that the computation based on Fig. 11 can be performed.

That is, the information processing device B 120 being the explanatory-variable retaining device does not need to output the secure data (explanatory variable) outward, and thus can perform the computation with the matrices X and V including the explanatory variable remaining intact input at step S101b.

In addition, the value (d) based on the outcome variable (y) being the secure data is used in (Expression d) illustrated in Fig. 11.

However, at step S103, the information processing device A 110 being the outcome-variable retaining device generates the computed result with the value (d) based on the outcome variable (y), namely, the arithmetic result (t_0) of the arithmetic expression 302 illustrated in Fig. 11 to input the arithmetic result (t_0) into the information processing device B 120.

Therefore, the information processing device B 120 is required only to substitute the input value (t_0) into (Expression d) of Fig. 11, and does not need to perform, as the secure computation, (Expression d) illustrated in Fig. 11.

The arithmetic expression 301 expressed in (Expression e) of Fig. 11 is the inner product (t_s) calculated at step S102, and thus only the value is applied with the value calculated with the secure computation at the previous step S102.

In this manner, the performance of the processing based on the flow illustrated in Fig. 10, makes a considerable reduction in processing requiring the secure computation and a considerable reduction in computational complexity required in the calculation processing of the logistic regression parameter β_i (i = 0, 1, ..., r), so that reduction in computational cost and enhanced speed in processing are made possible.

(Step S105)

Next, at step S105, the output unit 145 of the parameter-calculation execution unit 121 in the information processing device B 120 being the explanatory-variable (x) retaining device outputs the logistic regression parameter β_i (i = 0, 1, ..., r) calculated at step S104 to the data processing unit in the information processing device B 120.

The data processing unit in the information processing device B 120 substitutes the logistic regression parameter β_i (i = 0, 1, ..., r) output from the parameter-calculation execution unit 121, into the logistic regression model, namely, (Expression 1) described earlier, to perform processing of estimating the outcome variable (y) from various values of the explanatory variable (x).

As described earlier, in accordance with the logistic regression model expressed in (Expression 1), the probability p(x) of occurrence of the event can be calculated under the condition including the observation values (x_1, ..., x_r) of the explanatory variable (x) given.

The probability p(x) corresponds to the value of the outcome variable (y).

Note that, as interpreted from the flowchart illustrated in Fig. 10, the information processing device B 120, namely, the information processing device B 120 being the explanatory-variable (x) retaining device performs the calculation of the logistic regression parameter β_i (i = 0, 1, ..., r) in the exemplary processing.

The information processing device A 110 being the outcome-variable (y) retaining device does not perform the calculation of the logistic regression parameter $\beta\_i$ (i = 0, 1, ..., r).

The information processing device B 120 being the explanatory-variable (x) retaining device that has performed the calculation of the logistic regression parameter $\beta\_i$ (i = 0, 1, ..., r), can provide the calculated parameter to the information processing device A 110 in response to a request from the information processing device A 110 being the outcome-variable (y) retaining device. The logistic regression parameter $\beta\_i$ (i = 0, 1, ..., r) itself is not the secure data, and thus is allowed to be subjected to input/output processing or sharing processing between the devices.

In the processing based on the flow illustrated in Fig. 10, the computation in the secure computation processing includes only the computation of the inner product (t_s) of the explanatory variable (x) and the outcome variable (y).

That is, as described earlier, only the calculation processing of the inner product (t_s) based on (Expression 13) below, is included.

[Math. 23]

$$t_s = \sum_{i=1}^{n} x_s^i y_i = \sum_{j=1}^{J} x_{js} d_j$$

$$(s = 1, ..., r) \qquad\qquad ... \text{(Expression 13)}$$

The inner product (t_s) of the explanatory variable (x) and the outcome variable (y) expressed in (Expression 13) above is arithmetic including the explanatory variable (x) and the outcome variable (y) being the secure data not allowed to be released, and the arithmetic is required to be performed as the secure computation.

That is, for example, as described earlier with reference to Figs. 6 and 7, the converted data, such as the segmented data of each of the explanatory variable (x) and the outcome variable (y) being the secure data, is generated and then the arithmetic applied with the generated converted data is performed.

However, in the flow illustrated in Fig. 10, the processing requiring the secure computation includes only the calculation processing of the inner product (t_s) of the explanatory variable (x) and the outcome variable (y) at step S102.

That is, the secure computation of, for example, the matrix X and the matrix V required in the iterative computation of the Newton-Raphson method described earlier with reference to Fig. 8, is unnecessary to perform, and thus a considerable reduction is made in computational complexity required in the parameter calculation, so that reduction in computational cost and enhanced speed in processing are made possible.

[6. Reduction Effect in Computational Complexity of Parameter Calculation Processing according to Present Disclosure]

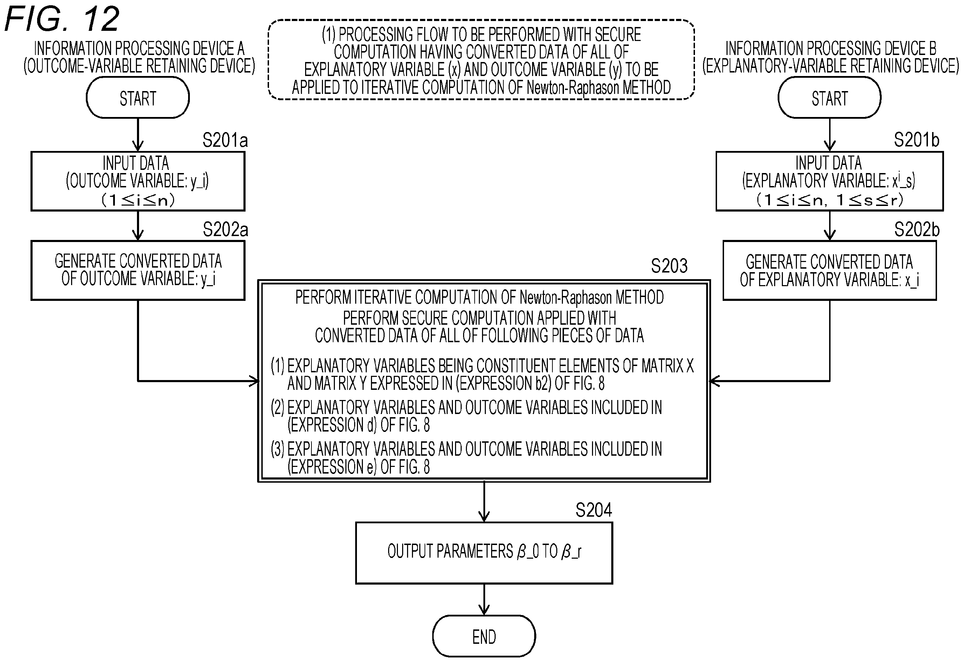

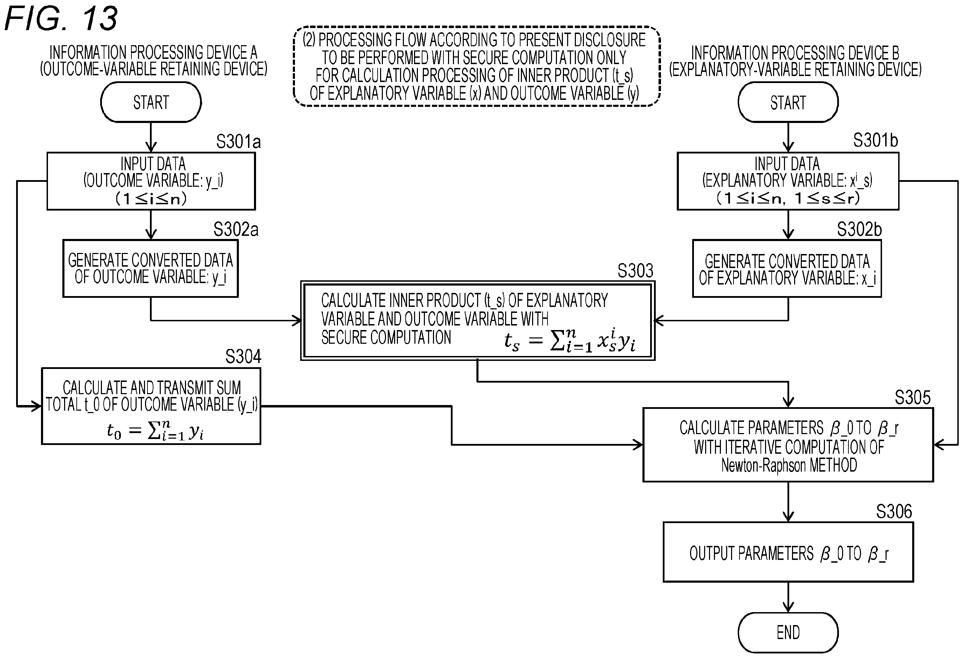

Next, a reduction effect in the computational complexity of the parameter calculation processing according to the present disclosure, will be described with reference to two flowcharts illustrated in Figs. 12 and 13.

Figs. 12 and 13 illustrate the following two flowcharts:

(1) a processing flow to be performed with the secure computation having the converted data of all of the explanatory variable (x) and the outcome variable (y) to be applied to the iterative computation of the Newton-Raphason method, and

(2) a processing flow according to the present disclosure to be performed with the secure computation only for the calculation processing of the inner product (t_s) of the explanatory variable (x) and the outcome variable (y).

The calculation sequence of the logistic regression parameter $\beta\_i$ (i = 0, 1, ..., r) based on each of the two processing flows, will be described.

First, "(1) the processing to be performed with the secure computation having the converted data of all of the explanatory variable (x) and the outcome variable (y) to be applied to the iterative computation of the Newton-Raphason method" will be described in accordance with the flowchart illustrated in Fig. 12.

(Steps S201a and S201b)

The processing at steps S201a and b includes the data input processing of the input units.

At step S201a, the information processing device A 110 being the outcome-variable (y) retaining device acquires the outcome variable y_i (note that, i = 1, ..., n) retained in the storage unit of the information processing device A 110, from the storage unit, to input the outcome variable y_i into the data processing unit (arithmetic execution unit) of the information processing device A 110.

Meanwhile, at step S201b, the information processing device B 120 being the explanatory variable (x) retaining device acquires the explanatory variables ($x^i\_1$, $x^i\_2$, ..., $x^i\_r$) (note that, i = 1, ..., n) retained in the storage unit of the information processing device B 120, from the storage unit, to input the explanatory variables ($x^i\_1$, $x^i\_2$, ..., $x^i\_r$) into the data processing unit (arithmetic execution unit).

(Steps S202a and S202b)

The processing at steps S202a and S202b includes the generation processing of the converted data of the secure data in the data processing units (arithmetic execution units) of the information processing device A 110 and the information processing device B 120.

The explanatory variable (x) and the outcome variable (y) both are the secure data subject to restriction of release, and thus the secure data is not allowed to be directly used in the calculation processing of the logistic regression parameter $\beta\_i$ (i = 0, 1, ..., r) .

Thus, the generation processing of the converted data of the explanatory variable (x) and the outcome variable (y) being the secure data is performed.

At step S202a, the information processing device A 110 being the outcome-variable retaining device generates the converted data of the outcome variable (y).

Meanwhile, at step S202b, the information processing device B 120 being the explanatory variable (x) retaining device generates the converted data of the explanatory variable (x).

Various modes of converted data, such as encrypted data of the secure data (explanatory variable (x) and outcome variable (y)) and the segmented data described with reference to Figs. 6 and 7, for example, are provided as the converted data.

(Step S203)

The next processing at step S203 includes the calculation processing of the logistic regression parameter $\beta\_i$ (i = 0, 1, ..., r) based on the maximum likelihood method with the Newton-Raphson method (iterative convergence method) described earlier with reference to Fig. 8.

As described earlier with reference to Fig. 8, in a case where the estimation processing of the logistic regression parameter is performed, (Expression a) illustrated in Fig. 8 is required to be repeatedly computed until (Expression a2) illustrated in Fig. 8 is satisfied.

However, as illustrated in Fig. 8, the explanatory variable (x) and the outcome variable (y) as the secure data are used in quantities in (Expression a).

The secure data, namely, the explanatory variable (x) and the outcome variable (y) individually retained by the two different information processing devices are not allowed to be released mutually.

Therefore, the iterative computation processing of (Expression a) illustrated in Fig. 8, until the satisfaction of (Expression a2), is required to be performed as the secure computation.

The secure computation needs processing of individually converting the secure data and making an input or output between the devices, for example, generation of the segmented data of the secure data and input or output of part of the segmented data between the devices as described with reference to Figs. 6 and 7.

For example, the matrix X and the matrix V expressed in (Expression b2) of Fig. 8 each include a large number of explanatory variables. Each of the explanatory variables is the secure data.

Therefore, in order to perform the secure computation, for example, processing of generating the converted data, such as the segmented data, for each of the explanatory variables included in the matrix X and the matrix V expressed in (Expression b2) of Fig. 8 and inputting or outputting the converted data between the devices is required.

For (Expression d) and (Expression e) illustrated in Fig. 8, similarly, there is a need to generate the converted data, such as the segmented data, individually for the explanatory variable (x) and the outcome variable (y) included as the constituent elements of the expressions, and input or output the converted data between the devices.

Such data conversion processing and data input/output processing increase as the amount of secure data to be applied to the secure computation increases.

Therefore, for a large amount of secure data, the iterative computation processing of (Expression a) illustrated in Fig. 8 needs a plenty of computational time and a plenty of computational resources. That is, the computational cost increases.

That is, the processing at step S203 illustrated in Fig. 12 needs a plenty of computational resources and a plenty of computational time.

(Step S204)

After the calculation of the logistic regression parameter $\beta\_i$ (i = 0, 1, ..., r) with the secure computation at step S203, the two information processing devices A and B next output the parameter to the data processing units at step S204.

The data processing units each perform, for example, processing of estimating an outcome variable from a new explanatory variable with the calculated parameter, in accordance with (Expression 1) described earlier, namely, the logistic regression model.

In the flow illustrated in Fig. 12, the calculation processing of the logistic regression parameter $\beta\_i$ (i = 0, 1, ..., r) based on the maximum likelihood method with the Newton-Raphson method (iterative convergence method) at step S203, is enormous in computational complexity.

This is because, as described earlier with reference to Fig. 8, there is a need to use a large amount of converted data of the explanatory variable (x) and the outcome variable (y) in a case where the parameter calculation processing with the Newton-Raphson method (iterative convergence method) illustrated in Fig. 8 is performed.

The matrix X and the matrix V expressed in (Expression b2) of Fig. 8 each include a large amount of explanatory variables. Each of the explanatory variables is the secure data.

For (Expression d) and (Expression e) illustrated in Fig. 8, similarly, all of the explanatory variable (x) and the outcome variable (y) included as the constituent elements of the expressions are the secure data.

Therefore, in a case where the computation of the expressions is performed, there is a need to perform computation processing with generation of the converted data, such as the segmented data, corresponding to each of the explanatory variables and the outcome variables being the secure data.

In this manner, the performance of the processing based on the flow illustrated in Fig. 12 increases the computational complexity of the generation processing of the converted data of the secure data and the computation processing with the converted data, and thus there is a problem that the computation processing resources and the computational time increase.

Next, the flow illustrated in Fig. 13, namely, "(2) the processing according to the present disclosure, to be performed with the secure computation only for the calculation processing of the inner product (t_s) of the explanatory variable (x) and the outcome variable (y)" will be described.

(Steps S301a and S301b)

The processing at steps S301a and b includes the data input processing of the input units.

At step S301a, the information processing device A 110 being the outcome-variable (y) retaining device acquires the outcome variable y_i (note that, i = 1, ..., n) retained in the storage unit of the information processing device A 110, from the storage unit, to input the outcome variable y_i into the data processing unit (arithmetic execution unit) of the information processing device A 110.

Meanwhile, at step S301b, the information processing device B 120 being the explanatory variable (x) retaining device acquires the explanatory variables $(x^i\_1, x^i\_2, ..., x^i\_r)$ (note that, i = 1, ..., n) retained in the storage unit of the information processing device B 120, from the storage unit, to input the explanatory variables $(x^i\_1, x^i\_2, ..., x^i\_r)$ into the data processing unit (arithmetic execution unit).

(Steps S302a and S302b)

The processing at steps S302a and S302b includes the generation processing of the converted data of the secure data in the data processing units (arithmetic execution units) of the information processing device A 110 and the information processing device B 120.

The explanatory variable (x) and the outcome variable (y) both are the secure data subject to restriction of release, and thus the secure data is not allowed to be directly used in the calculation processing of the logistic regression parameter $\beta\_i$ (i = 0, 1, ..., r) .

Thus, the generation processing of the converted data of the explanatory variable (x) and the outcome variable (y) being the secure data is performed.

At step S302a, the information processing device A 110 being the outcome-variable retaining device generates the converted data of the outcome variable (y).

Meanwhile, at step S302b, the information processing device B 120 being the explanatory variable (x) retaining device generates the converted data of the explanatory variable (x).

Various modes of converted data, such as encrypted data of the secure data (explanatory variable (x) and outcome variable (y)) and the segmented data described with reference to Figs. 6 and 7, for example, are provided as the converted data.

(Step S303)

The processing at step S303 includes the calculation processing of the inner product (t_s) of the explanatory variable (x) and the outcome variable (y) in the data processing units (arithmetic execution units) of the information processing device A 110 and the information processing device B 120.

The processing corresponds to the processing at step S102 in the flow of Fig. 10 described earlier.

As described earlier, the inner product (t_s) of the explanatory variable (x) and the outcome variable (y) is calculated in accordance with (Expression 12) below.

[Math. 24]

$$t_s = \sum_{i=1}^{n} x_s^i y_i \quad (s = 1, \dots, r) \qquad \dots \text{(Expression 12)}$$

Note that, as described above, the inner product (t_s) of the explanatory variable (x) and the outcome variable (y) expressed in (Expression 12) above, can be expressed in (Expression 13) below including (d) in (2) the profile unit data illustrated on the right of Fig. 5 described earlier with reference to Fig. 5, namely, the data (d) corresponding to the number of samples having the outcome variable (y) satisfying y = 1

[Math. 25]

$$t_s = \sum_{i=1}^{n} x_s^i y_i = \sum_{j=1}^{J} x_{js} d_j$$

$$(s = 1, \dots, r) \qquad \dots \text{(Expression 13)}$$

As described with reference to Fig. 11, the arithmetic expression applied with the data d, for calculating the inner product (t_s) of the explanatory variable (x) and the outcome variable (y) in (Expression 13) above, corresponds to the arithmetic expression 301 in (Expression e) in Fig. 11.

Because the explanatory variable (x) and the outcome variable (y) both are the secure data subject to restriction of release, the calculation processing of the inner product (t_s) based on (Expression 12) above is required to be performed with arithmetic not applied directly with the explanatory variable (x) and the outcome variable (y) being the secure data, namely, the secure computation as described with reference to Figs. 6 and 7.

The converted data of the secure data (explanatory variable (x) and outcome variable (y)) generated at steps S302a and S302b, is used for the secure computation.

In the flow illustrated in Fig. 13, the secure computation with the converted data of the secure data (explanatory variable (x) and outcome variable (y)) is used only for the processing at step S303.

Only the computation processing of part of (Expression e) described earlier with reference to Fig. 11, is performed as the secure computation.

Similarly to the flow illustrated in Fig. 12, the parameter calculation processing with the Newton-Raphson method (iterative convergence method) described with reference to Figs. 8 and 11, is performed in the flow illustrated in Fig. 13.

In the flow illustrated in Fig. 12, all of the computation of the matrix X and the matrix V expressed in (Expression b2) of Fig. 8 and the computation including the explanatory variable (x) and the outcome variable (y) in (Expression d) and (Expression e) are performed as the secure computation. That is, the computation processing is performed with the generation of the converted data, such as the segmented data, corresponding to each of the explanatory variables and the outcome variables.

However, in the processing based on the flow illustrated in Fig. 13, only the calculation of the arithmetic expression 301 in (Expression e) illustrated in Fig. 11 is performed as the secure computation.

(Step S304)

The next processing at step S304 is that the information processing device A 110 being the outcome-variable (y) retaining device calculates the sum total (t_0) of the outcome variable (y) in accordance with (Expression 14) below to output the calculated value to the parameter-calculation execution unit 121 of the information processing device B120 through the data transmission/reception unit 134.

[Math. 26]

$$t_0 = \sum_{i=1}^{n} y_i$$

... (Expression 14)

Note that, the sum total ($t\_0$) of the outcome variable (y) expressed in (Expression 14) above can be expressed in (Expression 15) below including (d) in (2) the profile unit data illustrated on the right of Fig. 5 described earlier with reference to Fig. 5, namely, the data (d) corresponding to the number of samples having the outcome variable (y) satisfying y = 1.

[Math. 27]

$$t_0 = \sum_{i=1}^{n} y_i = \sum_{j=1}^{J} d_j$$

... (Expression 15)

The arithmetic applied with d expressed in (Expression 15) above, namely, the arithmetic expression applied with the data d corresponding to the number of samples having the outcome variable (y) satisfying y = 1, is included in part of (Expression d) expressed in the computational expression for estimating the parameter β in accordance with the maximum likelihood method with the Newton-Raphson method (iterative convergence method) described earlier with reference to Fig. 8.

As illustrated in Fig. 11, the arithmetic expression applied with the data d, for calculating the sum total ($t\_0$) of the outcome variable (y) in (Expression 15) above, corresponds to the arithmetic expression 302 in (Expression d) in Fig. 11.

The calculation processing of the sum total ($t\_0$) of the outcome variable (y), to be performed at step S304, corresponds to the processing of performing the arithmetic expression 302 in (Expression d) in Fig. 11.

Note that, the processing at step S304 is performed inside the information processing device A 110 being the outcome-variable (y) retaining device, and thus the processing is not required to be performed as the secure computation.

That is, without performance of generation processing of the converted data of the outcome variable (y) and output processing of the converted data to the external device, the processing at step S304 can be performed to calculate the sum total ($t\_0$) of the outcome variable (y) in the arithmetic device inside the information processing device A 110 with acquisition of the outcome variable (y) being the secure data retained inside the information processing device A 110 and application of the acquired outcome variable (y) remaining intact.

In this manner, the typical arithmetic processing applied with the secure data, instead of the secure computation, can make a considerable reduction in computational time or computational resources in comparison to performance of the secure computation.

The information processing device A 110 calculates the sum total ($t\_0$) of the outcome variable (y) in accordance with (Expression 14) or (Expression 15) described above to output the calculated value to the information processing device B 120. The sum total ($t\_0$) of the outcome variable (y) itself is not the secure data, and thus can be output outward.

(Step S305)

Next, at step S305, the information processing device B 120 being the explanatory variable (x) retaining device performs the iterative computation of the Newton-Raphson method described earlier with reference to Figs. 8 and 11, to the expression based on the logistic regression model expressed in (Expression 1) described earlier, to perform the updating and calculation processing of the logistic regression parameter $\beta\_i$ (i = 0, 1, ..., r).

(Step S306)

Next, at step S306, the information processing device B 120 being the explanatory variable (x) retaining device, outputs the logistic regression parameter $\beta\_i$ (i = 0, 1, ..., r) calculated at step S305, to the data processing unit of the information processing device B 120.

The data processing unit of the information processing device B 120 substitutes the logistic regression parameter $\beta\_i$ (i = 0, 1, ..., r) into the logistic regression model, namely, (Expression 1) described earlier, to perform the processing of estimating the outcome variable (y) from various values of the explanatory variable (x).

Note that, the information processing device B 120 being the explanatory variable (x) retaining device that has performed the calculation of the logistic regression parameter $\beta\_i$ ($i = 0, 1, ..., r$) provides the calculated parameter to the information processing device A 110 in response to a request from the information processing device A 110 being the outcome-variable (y) retaining device. The logistic regression parameter $\beta\_i$ ($i = 0, 1, ..., r$) itself is not the secure data, and thus is allowed to be subjected to the input/output processing or the sharing processing between the devices.

In the processing based on the flow illustrated in Fig. 13, the computation in the secure computation processing includes only the computation of the inner product ($t\_s$) of the explanatory variable (x) and the outcome variable (y) to be performed at step S303.

At step S305 in the flow described with reference to Fig. 13, for example, the matrix X and the matrix V are computed in the iterative computation of the Newton-Raphson method illustrated in Figs. 8 and 11. The matrices each include the explanatory variable (x) being the secure data.

However, because the processing at step S305 is performed in the information processing device B being the explanatory-variable retaining device, the secure data (explanatory variable) is not required to be output outward, so that the computation can be performed with the matrices X and V including the explanatory variable remaining intact input at step S101b.

In addition, the value (d) based on the outcome variable (y) being the secure data is used in (Expression d) illustrated in Fig. 11.

However, the information processing device A being the outcome-variable retaining device generates, at step S304, the computed result with the value (d) based on the outcome variable (y), namely, the arithmetic result of the arithmetic expression 302 illustrated in Fig. 11, and the information processing device B receives the arithmetic result and can use the arithmetic result remaining intact, so that no secure computation is required to be performed for (Expression d) illustrated in Fig. 11.

In this manner, the performance of the processing based on the flow illustrated in Fig. 13 makes a considerable reduction in processing requiring the secure computation and a considerable reduction in computational complexity required in the calculation processing of the logistic regression parameter $\beta\_i$ ($i = 0, 1, ..., r$), so that reduction in computational cost and enhanced speed in processing are made possible.

[7. Exemplary Hardware Configuration of Information Processing Device]

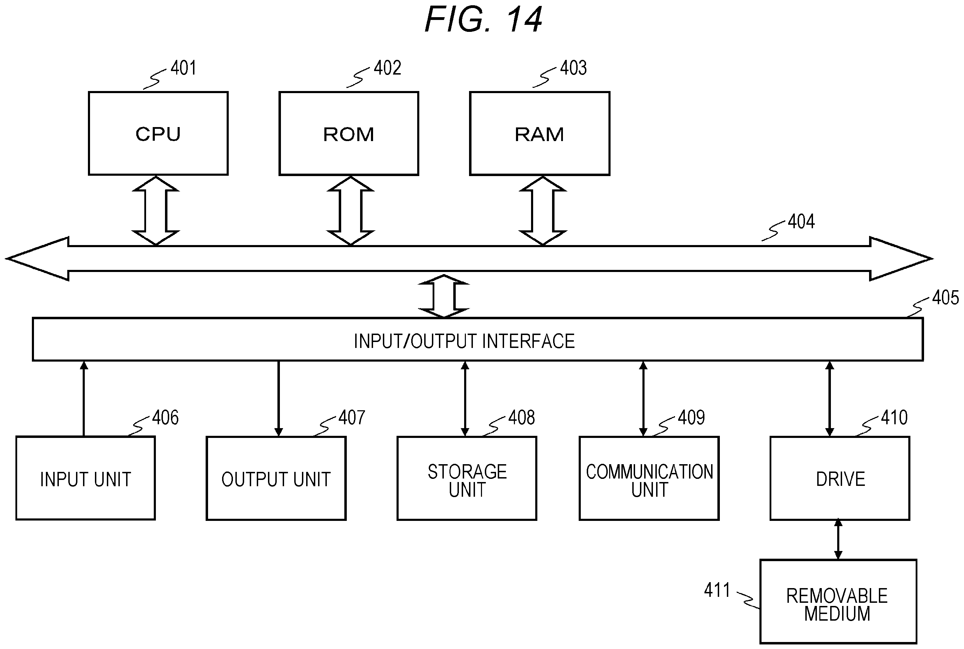

Finally, an exemplary hardware configuration of an information processing device that performs the processing according to the embodiment, will be described with reference to Fig. 14.

Fig. 14 is a diagram of the exemplary hardware configuration of the information processing device.

A central processing unit (CPU) 401 functions as a control unit or a data processing unit that performs various types of processing in accordance with a program stored in a read only memory (ROM) 402 or a storage unit 408. For example, the CPU 401 performs the processing based on the sequence described in the embodiment. A random access memory (RAM) 403 stores, for example, the program to be performed by the CPU 401 and data. The CPU 401, the ROM 402, and the RAM 403 are mutually connected through a bus 404.

The CPU 401 is connected to an input/output interface 405 through the bus 404, and the input/output interface 405 is connected with an input unit 406 including various switches, a keyboard, a mouse, a microphone, and the like and an output unit 407 including a display, a speaker, and the like. The CPU 401 performs the various types of processing in response to a command input from the input unit 406 to output a processing result to, for example, the output unit 407.

The storage unit 408 connected to the input/output interface 405 includes, for example, a hard disk and the like, and stores the program to be performed by the CPU 401 and various types of data. A communication unit 409 functions as a transmission/reception unit for data communication through a network, such as the Internet or a local area network, and communicates with an external device.

A drive 410 connected to the input/output interface 405 drives a removable medium 411 such as a magnetic disk, an optical disc, a magneto-optical disc, or a semiconductor memory, such as a memory card, to perform recording or reading of data.

[8. Summary of Configuration of Present Disclosure]

The embodiment of the present disclosure has been described in detail above with reference to the specific embodiment. However, it is obvious that a person skilled in the art may make alterations or replacements to the embodiment without departing from the scope of the present disclosure. That is, the present invention has been disclosed in an exemplified mode, and thus the present invention should not be interpreted in a limited way. The scope of the claims should be considered.

In addition, the set of processing described in the present specification can be performed by hardware, software, or a combined configuration of the two. In a case where the processing is performed by the software, a program including a processing sequence recorded is installed into a memory in a computer built in dedicated hardware or the program is installed into a general-purpose computer capable of performing various types of processing, so that the processing can be performed. For example, the program can be previously recorded in a recording medium. In addition to installation from the recording medium into a computer, the program received through a network, such as a local area network (LAN) or the Internet, can be installed into a built-in recording medium, such as a hard disk.

Note that, the various types of processing described in the specification may be performed in parallel or individually in response to the throughput of a device that performs the processing or as necessary, in addition to being performed on a time series basis in accordance with the description. In addition, a system in the present specification is a logical aggregate configuration including a plurality of devices, but is not limited to a configuration including the constituent devices in the same housing.

INDUSTRIAL APPLICABILITY

As described above, according to the configuration of one embodiment of the present disclosure, high-speed and efficient parameter calculation processing of a logistic regression model is achieved.

Specifically, a logistic regression parameter is calculated, the logistic regression parameter being a parameter of the logistic regression model indicating the relationship between an explanatory variable and an outcome variable being secure data corresponding to each sample. A data processing unit calculates the inner product (t_s) of the explanatory variable and the outcome variable with application of secure computation being computation processing applied with converted data of each of the variables, and performs computation processing excluding the calculation processing of the inner product, as computation processing without the converted data, to calculate the logistic regression parameter in accordance with the maximum likelihood method with the Newton-Raphson method (iterative convergence method).

According to the present configuration, the high-speed and efficient parameter calculation processing of the logistic regression model is achieved.

REFERENCE SINGS LIST

| | |
|---|---|
| 110 | Information processing device A |
| 111 | Parameter-calculation execution unit |
| 112 | Inner-product computation unit |
| 113 | Iterative-computation input-value generation unit |
| 114 | Data transmission/reception unit |
| 120 | Information processing device B |
| 121 | Input unit |
| 122 | Inner-product computation unit |
| 123 | Data transmission/reception unit |
| 124 | Iterative computation unit |
| 125 | Output unit |
| 401 | CPU |
| 402 | ROM |
| 403 | RAM |
| 404 | Bus |
| 405 | Input/output interface |
| 406 | Input unit |
| 407 | Output unit |
| 408 | Storage unit |
| 409 | Communication unit |
| 410 | Drive |
| 411 | Removable medium |

**Claims**

1. An information processing device comprising:

a data processing unit configured to calculate a logistic regression parameter being a parameter of a logistic regression model indicating a relationship between a first variable and a second variable being two different types of secure data associated with each of a plurality of samples, wherein the first variable is an explanatory variable, and the second variable is an outcome variable, wherein the information processing device is a retaining device of the explanatory variable,

wherein the data processing unit calculates an inner product (t_s) of the first variable and the second variable with application of secure computation being computation processing applied with converted data of each of the variables, and

performs computation processing excluding the calculation processing of the inner product, as computation processing without the converted data, to calculate the logistic regression parameter, wherein the data processing unit receives a computed result applied with the outcome variable from an outcome-variable retaining device, and calculates the logistic regression parameter with the computed result applied with the received outcome variable, wherein the computed result applied with the outcome variable is a sum total (t_0) of the outcome variable.

2. The information processing device according to claim 1, wherein the data processing unit calculates the logistic regression parameter in accordance with a maximum likelihood method with a Newton-Raphson method.

3. The information processing device according to claim 1, wherein the data processing unit performs the calculation processing of the inner product (t_s) of the explanatory variable and the outcome variable with the secure computation applied with segmented data of the explanatory variable and segmented data of the outcome variable.

4. The information processing device according to claim 1, wherein the information processing device is a retaining device of the explanatory variable, and
the data processing unit performs the computation processing excluding the calculation processing of the inner product, applied with the explanatory variable, as computation processing applied with the explanatory variable remaining intact, without the application of the secure computation, in the calculation processing of the logistic regression parameter based on a maximum likelihood method with a Newton-Raphson method.

5. The information processing device according to claim 1, wherein the information processing device is a retaining device of the explanatory variable, and
the data processing unit outputs the logistic regression parameter calculated to an outcome-variable retaining device.

6. An information processing system comprising:

an explanatory-variable retaining device retaining an explanatory variable being secure data associated with each of a plurality of samples; and
an outcome-variable retaining device retaining an outcome variable being secure data associated with each of the plurality of samples,
wherein the outcome-variable retaining device calculates and outputs a sum total (t_0) of the outcome variable associated with each of the plurality of samples to the explanatory-variable retaining device,
the explanatory-variable retaining device includes a data processing unit configured to calculate a logistic regression parameter being a parameter of a logistic regression model indicating a relationship with the outcome variable, and
the data processing unit calculates an inner product (t_s) of the explanatory variable and the outcome variable, with application of secure computation being computation processing applied with converted data of each of the variables, and
calculates the logistic regression parameter with application of the inner product (t_s) calculated and the sum total (t_0) of the outcome variable input from the outcome-variable retaining device.

7. The information processing system according to claim 6, wherein the data processing unit calculates the logistic regression parameter in accordance with a maximum likelihood method with a Newton-Raphson method.

8. The information processing system according to claim 6, wherein the data processing unit performs the calculation processing of the inner product (t_s) of the explanatory variable and the outcome variable, with the secure computation applied with segmented data of the explanatory variable and segmented data of the outcome variable.

9. The information processing system according to claim 6, wherein the data processing unit performs computation processing excluding the calculation processing of the inner product, applied with the explanatory variable, as computation processing applied with the explanatory variable remaining intact, without the application of the secure computation, in the calculation processing of the logistic regression parameter based on a maximum likelihood method with a Newton-Raphson method.

10. An information processing method to be performed in an information processing device including

a data processing unit configured to calculate a logistic regression parameter being a parameter of a logistic regression model indicating a relationship between a first variable and a second variable being two different types of secure data associated with each of a plurality of samples, wherein the first variable is an explanatory variable, and the second variable is an outcome variable, wherein the information processing device is a retaining device of the explanatory variable, the information processing method comprising:

calculating, by the data processing unit, an inner product ($t\_s$) of the first variable and the second variable with application of secure computation being computation processing applied with converted data of each of the variables; and

calculating the logistic regression parameter with performance of computation processing excluding the calculation processing of the inner product, as computation processing without the converted data, wherein the data processing unit receives a computed result applied with the outcome variable from an outcome-variable retaining device, and calculates the logistic regression parameter with the computed result applied with the received outcome variable, wherein the computed result applied with the outcome variable is a sum total ($t\_0$) of the outcome variable.

11. An information processing method to be performed in an information processing system including:

an explanatory-variable retaining device retaining an explanatory variable being secure data associated with each of a plurality of samples; and

an outcome-variable retaining device retaining an outcome variable being secure data associated with each of the plurality of samples, the information processing method comprising:

calculating and outputting, by the outcome-variable retaining device, a sum total ($t\_0$) of the outcome variable associated with each of the plurality of samples to the explanatory-variable retaining device; and by a data processing unit included in the explanatory-variable retaining device, configured to calculate a logistic regression parameter being a parameter of a logistic regression model indicating a relationship with the outcome variable,

calculating an inner product ($t\_s$) of the explanatory variable and the outcome variable with application of secure computation being computation processing applied with converted data of each of the variables and

calculating the logistic regression parameter with application of the inner product ($t\_s$) calculated and the sum total ($t\_0$) of the outcome variable input from the outcome-variable retaining device.

12. A program for causing information processing to be executed in an information processing device including a data processing unit configured to calculate a logistic regression parameter being a parameter of a logistic regression model indicating a relationship between a first variable and a second variable being two different types of secure data associated with each of a plurality of samples, wherein the first variable is an explanatory variable, and the second variable is an outcome variable, wherein the information processing device is a retaining device of the explanatory variable, the program causing the data processing unit to execute:

processing of calculating an inner product ($t\_s$) of a first variable and a second variable with application of secure computation being computation processing applied with converted data of each of the variables; and processing of calculating the logistic regression parameter with performance of computation processing excluding the processing of calculating the inner product, as computation processing without the converted data, wherein the data processing unit receives a computed result applied with the outcome variable from an outcome-variable retaining device, and calculates the logistic regression parameter with the computed result applied with the received outcome variable, wherein the computed result applied with the outcome variable is a sum total ($t\_0$) of the outcome variable.

**Patentansprüche**

1. Informationsverarbeitungsvorrichtung, umfassend:

eine Datenverarbeitungseinheit, die dafür ausgelegt ist, einen Parameter der logistischen Regression zu berechnen, der ein Parameter eines logistischen Regressionsmodells ist, das eine Beziehung zwischen einer ersten Variablen und einer zweiten Variablen anzeigt, die zwei verschiedene Arten von sicheren Daten sind, die mit jeder von mehreren Proben verbunden sind,

wobei die erste Variable eine Erklärungsvariable und die zweite Variable eine Ergebnisvariable ist, wobei die Informationsverarbeitungsvorrichtung eine Speichervorrichtung für Erklärungsvariablen ist,

wobei die Datenverarbeitungseinheit ein inneres Produkt (t_s) der ersten Variablen und der zweiten Variablen unter Anwendung einer sicheren Berechnung, die eine Berechnungsverarbeitung mit umgewandelten Daten jeder der Variablen ist, berechnet und Berechnungsverarbeitung mit Ausnahme der Berechnungsverarbeitung des inneren Produkts als Berechnungsverarbeitung ohne die umgewandelten Daten durchführt, um den Parameter der logistischen Regression zu berechnen, wobei die Datenverarbeitungseinheit ein berechnetes Ergebnis, das mit der Ergebnisvariablen angewandt wird, von einer Ergebnisvariablen-Speichervorrichtung empfängt und den Parameter der logistischen Regression mit dem berechneten Ergebnis, das mit der empfangenen Ergebnisvariablen angewandt wird, berechnet, wobei das berechnete Ergebnis, das mit der Ergebnisvariablen angewandt wird, eine Gesamtsumme (t_0) der Ergebnisvariablen ist.

2. Informationsverarbeitungsvorrichtung gemäß Anspruch 1, wobei die Datenverarbeitungseinheit den Parameter der logistischen Regression entsprechend einem Größte-Wahrscheinlichkeit-Verfahren mit einem Newton-Raphson-Verfahren berechnet.

3. Informationsverarbeitungsvorrichtung gemäß Anspruch 1, wobei die Datenverarbeitungseinheit die Berechnungsverarbeitung des inneren Produkts (t_s) der Erklärungsvariablen und der Ergebnisvariablen mit der sicheren Berechnung durchführt, die mit segmentierten Daten der Erklärungsvariablen und segmentierten Daten der Ergebnisvariablen angewandt wird.

4. Informationsverarbeitungsvorrichtung gemäß Anspruch 1, wobei die Informationsverarbeitungsvorrichtung eine Speichervorrichtung für Erklärungsvariablen ist, und die Datenverarbeitungseinheit die Berechnungsverarbeitung mit Ausnahme der Berechnungsverarbeitung des inneren Produkts, angewandt mit der Erklärungsvariablen, als Berechnungsverarbeitung, bei der die Erklärungsvariable unverändert bleibt, ohne Anwendung der sicheren Berechnung in der Berechnungsverarbeitung des Parameters der logistischen Regression basierend auf einem Größte-Wahrscheinlichkeit-Verfahren mit einem Newton-Raphson-Verfahren durchführt.

5. Informationsverarbeitungsvorrichtung gemäß Anspruch 1, wobei die Informationsverarbeitungsvorrichtung eine Speichervorrichtung für Erklärungsvariablen ist, und die Datenverarbeitungseinheit den berechneten Parameter der logistischen Regression an eine Ergebnisvariablen-Speichervorrichtung ausgibt.

6. Informationsverarbeitungssystem, umfassend:

eine Erklärungsvariablen-Speichervorrichtung, die eine Erklärungsvariable speichert, welche sichere Daten darstellt, die mit jeder von mehreren Proben verknüpft sind; und

eine Ergebnisvariablen-Speichervorrichtung, die eine Ergebnisvariable speichert, welche sichere Daten darstellt, die mit jeder der mehreren Proben verknüpft sind,

wobei die Ergebnisvariablen-Speichervorrichtung eine Gesamtsumme (t_0) der Ergebnisvariablen, die mit jeder der mehreren Proben verknüpft ist, berechnet und an die Erklärungsvariablen-Speichervorrichtung ausgibt,

die Erklärungsvariablen-Speichervorrichtung eine Datenverarbeitungseinheit aufweist, die dafür ausgelegt ist, einen Parameter der logistischen Regression zu berechnen, der ein Parameter eines logistischen Regressionsmodells ist, das eine Beziehung zu der Ergebnisvariablen anzeigt, und

die Datenverarbeitungseinheit ein inneres Produkt (t_s) der Erklärungsvariablen und der Ergebnisvariablen unter Anwendung einer sicheren Berechnung, die eine Berechnungsverarbeitung mit umgewandelten Daten jeder der Variablen ist, berechnet und

den Parameter der logistischen Regression unter Anwendung des berechneten inneren Produkts (t_s) und der Gesamtsumme (t_0) der Ergebnisvariablen, die von der Ergebnisvariablen-Speichervorrichtung eingegeben werden, berechnet.

7. Informationsverarbeitungssystem gemäß Anspruch 6, wobei die Datenverarbeitungseinheit den Parameter der logistischen Regression entsprechend einem Größte-Wahrscheinlichkeit-Verfahren mit einem Newton-Raphson-Verfahren berechnet.

8. Informationsverarbeitungssystem gemäß Anspruch 6, wobei die Datenverarbeitungseinheit die Berechnungsverarbeitung des inneren Produkts (t_s) der Erklärungsvariablen und der Ergebnisvariablen mit der sicheren Berechnung durchführt, die mit segmentierten Daten der Erklärungsvariablen und segmentierten Daten der Ergebnisvariablen angewandt wird.

9. Informationsverarbeitungssystem gemäß Anspruch 6, wobei die Datenverarbeitungseinheit eine Berechnungsverarbeitung mit Ausnahme der Berechnungsverarbeitung des inneren Produkts, angewandt mit der erklärenden Variablen, als Berechnungsverarbeitung, bei der die erklärende Variable unverändert bleibt, ohne Anwendung der sicheren Berechnung in der Berechnungsverarbeitung des Parameters der logistischen Regression basierend auf einem Größte-Wahrscheinlichkeit-Verfahren mit einem Newton-Raphson-Verfahren durchführt.

10. Informationsverarbeitungsverfahren, das in einer Informationsverarbeitungsvorrichtung durchzuführen ist und beinhaltet:

   eine Datenverarbeitungseinheit, die dafür ausgelegt ist, einen Parameter der logistischen Regression zu berechnen, der ein Parameter eines logistischen Regressionsmodells ist, das eine Beziehung zwischen einer ersten Variablen und einer zweiten Variablen anzeigt, die zwei verschiedene Arten von sicheren Daten sind, die mit jeder von mehreren Proben verbunden sind,
   wobei die erste Variable eine Erklärungsvariable und die zweite Variable eine Ergebnisvariable ist, wobei die Informationsverarbeitungsvorrichtung eine Speichervorrichtung für Erklärungsvariablen ist, wobei das Informationsverarbeitungsverfahren umfasst:

   Berechnen, durch die Datenverarbeitungseinheit, eines inneren Produkts (t_s) der ersten und der zweiten Variablen unter Anwendung einer sicheren Berechnung, die eine Berechnungsverarbeitung mit umgewandelten Daten jeder der Variablen ist; und
   Berechnen des Parameters der logistischen Regression unter Durchführung einer Berechnungsverarbeitung mit Ausnahme der Berechnungsverarbeitung des inneren Produkts als Berechnungsverarbeitung ohne die umgewandelten Daten, wobei die Datenverarbeitungseinheit ein mit der Ergebnisvariablen angewandtes Berechnungsergebnis von einer Ergebnisvariablen-Speichervorrichtung empfängt und den Parameter der logistischen Regression mithilfe des mit der empfangenen Ergebnisvariablen angewandten Berechnungsergebnisses berechnet, wobei das mit der Ergebnisvariablen angewandte Berechnungsergebnis eine Gesamtsumme (t_0) der Ergebnisvariablen ist.

11. Informationsverarbeitungsverfahren, das in einem Informationsverarbeitungssystem durchzuführen ist und beinhaltet:

   eine Erklärungsvariablen-Speichervorrichtung, die eine Erklärungsvariable speichert, welche sichere Daten darstellt, die mit jeder von mehreren Proben verknüpft sind; und
   eine Ergebnisvariablen-Speichervorrichtung, die eine Ergebnisvariable speichert, welche sichere Daten darstellt, die mit jeder der mehreren Proben verknüpft sind,
   wobei das Informationsverarbeitungsverfahren umfasst:

   Berechnen, durch die Ergebnisvariablen-Speichervorrichtung, einer Gesamtsumme (t_0) der Ergebnisvariablen, die mit jeder der mehreren Proben verknüpft ist, und Ausgeben an die Erklärungsvariablen-Speichervorrichtung; und
   durch eine Datenverarbeitungseinheit, die in der Erklärungsvariablen-Speichervorrichtung enthalten und dafür ausgelegt ist, einen Parameter der logistischen Regression, der ein Parameter eines logistischen Regressionsmodells ist, das eine Beziehung zur Ergebnisvariablen anzeigt, zu berechnen,

   Berechnen eines inneren Produkts (t_s) der Erklärungsvariablen und der Ergebnisvariablen unter Anwendung einer sicheren Berechnung, die eine Berechnungsverarbeitung mit umgewandelten Daten jeder der Variablen ist, und
   Berechnen des Parameters der logistischen Regression unter Anwendung des berechneten inneren Produkts (t_s) und der Gesamtsumme (t_0) der Ergebnisvariablen, die von der Ergebnisvariablen-Speichervorrichtung eingegeben wird.

12. Programm zum Veranlassen der Ausführung einer Informationsverarbeitung in einer Informationsverarbeitungsvorrichtung, die eine Datenverarbeitungseinheit aufweist, die dafür ausgelegt ist, einen Parameter der logistischen Regression zu berechnen, der ein Parameter eines logistischen Regressionsmodells ist, das eine Beziehung zwischen einer ersten Variablen und einer zweiten Variablen anzeigt, die zwei verschiedene Arten von sicheren Daten sind, die mit jeder von mehreren Proben verknüpft sind, wobei die erste Variable eine Erklärungsvariable und die zweite Variable eine Ergebnisvariable ist, wobei die Informationsverarbeitungsvorrichtung eine Speichervorrichtung für Erklärungsvariablen ist, wobei das Programm bewirkt, dass die Datenverarbeitungseinheit Folgendes ausführt:

Verarbeiten der Berechnung eines inneren Produkts (t_s) einer ersten Variablen und einer zweiten Variablen unter Anwendung einer sicheren Berechnung, die eine Berechnungsverarbeitung mit umgewandelten Daten jeder der Variablen ist; und

Verarbeiten der Berechnung des Parameters der logistischen Regression unter Durchführung einer Berechnungsverarbeitung mit Ausnahme der Berechnungsverarbeitung des inneren Produkts als Berechnungsverarbeitung ohne die umgewandelten Daten, wobei die Datenverarbeitungseinheit ein berechnetes Ergebnis, das mit der Ergebnisvariablen angewandt wird, von einer Ergebnisvariablen-Speichervorrichtung empfängt und den Parameter der logistischen Regression mit dem berechneten Ergebnis, das mit der empfangenen Ergebnisvariablen angewandt wird, berechnet, wobei das berechnete Ergebnis, das mit der Ergebnisvariablen angewandt wird, eine Gesamtsumme (t_0) der Ergebnisvariablen ist.

## Revendications

1. Dispositif de traitement d'informations comprenant :

une unité de traitement de données configurée pour calculer un paramètre de régression logistique étant un paramètre d'un modèle de régression logistique indiquant une relation entre une première variable et une seconde variable étant deux types différents de données sécurisées associées à chacun d'une pluralité d'échantillons,

où la première variable est une variable explicative et la seconde variable est une variable de résultat, où le dispositif de traitement d'informations est un dispositif de retenue de la variable explicative,

où l'unité de traitement de données calcule un produit interne (t_s) de la première variable et de la seconde variable, l'application d'un calcul sécurisé étant un traitement de calcul appliqué avec des données converties de chacune des variables, et

effectue un traitement de calcul en excluant le traitement de calcul du produit interne, en tant que traitement de calcul sans les données converties, pour calculer le paramètre de régression logistique, où l'unité de traitement de données reçoit un résultat calculé appliqué avec la variable de résultat d'un dispositif de retenue de variable de résultat, et calcule le paramètre de régression logistique avec le résultat calculé appliqué avec la variable de résultat reçue, où le résultat calculé appliqué avec la variable de résultat est une somme totale (t_0) de la variable de résultat.

2. Dispositif de traitement d'informations selon la revendication 1, dans lequel l'unité de traitement de données calcule le paramètre de régression logistique selon une méthode de vraisemblance maximale avec une méthode de Newton-Raphson.

3. Dispositif de traitement d'informations selon la revendication 1, dans lequel l'unité de traitement de données effectue le traitement de calcul du produit interne (t_s) de la variable explicative et de la variable de résultat, le calcul sécurisé étant appliqué avec des données segmentées de la variable explicative et des données segmentées de la variable de résultat.

4. Dispositif de traitement d'informations selon la revendication 1, dans lequel le dispositif de traitement d'informations est un dispositif de retenue de la variable explicative, et

l'unité de traitement de données effectue le traitement de calcul en excluant le traitement de calcul du produit interne, appliqué avec la variable explicative, en tant que traitement de calcul appliqué avec la variable explicative restant intacte, sans l'application du calcul sécurisé, dans le traitement de calcul du paramètre de régression logistique basé sur une méthode de vraisemblance maximale avec une méthode de Newton-Raphson.

5. Dispositif de traitement d'informations selon la revendication 1, dans lequel le dispositif de traitement d'informations est un dispositif de retenue de la variable explicative, et

l'unité de traitement de données délivre en sortie le paramètre de régression logistique calculé à un dispositif de retenue de variable de résultat.

**6.** Système de traitement d'informations comprenant :

un dispositif de retenue de variable explicative retenant une variable explicative étant des données sécurisées associées à chacun d'une pluralité d'échantillons ; et

un dispositif de retenue de variable de résultat retenant une variable de résultat étant des données sécurisées associées à chacun de la pluralité d'échantillons,

où le dispositif de retenue de variable de résultat calcule et délivre en sortie au dispositif de retenue de variable explicative une somme totale (t_0) de la variable de résultat associée à chacun de la pluralité d'échantillons,

le dispositif de retenue de variable explicative comprend une unité de traitement de données configurée pour calculer un paramètre de régression logistique étant un paramètre d'un modèle de régression logistique indiquant une relation avec la variable de résultat, et

l'unité de traitement de données calcule un produit interne (t_s) de la variable explicative et de la variable de résultat, l'application d'un calcul sécurisé étant un traitement de calcul appliqué avec des données converties de chacune des variables, et

calcule le paramètre de régression logistique avec l'application du produit interne (t_s) calculé et de la somme totale (t_0) de la variable de résultat entrée depuis le dispositif de retenue de variable de résultat.

**7.** Système de traitement d'informations selon la revendication 6, dans lequel l'unité de traitement de donnée calcule le paramètre de régression logistique selon une méthode de vraisemblance maximale avec une méthode de Newton-Raphson.

**8.** Système de traitement d'informations selon la revendication 6, dans lequel l'unité de traitement de données effectue le traitement de calcul du produit interne (t_s) de la variable explicative et de la variable de résultat, le calcul sécurisé étant appliqué avec des données segmentées de la variable explicative et des données segmentées de la variable de résultat.

**9.** Système de traitement d'informations selon la revendication 6, dans lequel l'unité de traitement de données effectue un traitement de calcul excluant le traitement de calcul du produit interne, appliqué avec la variable explicative, en tant que traitement de calcul appliqué avec la variable explicative restant intacte, sans l'application du calcul sécurisé, dans le traitement de calcul du paramètre de régression logistique basé sur une méthode de vraisemblance maximale avec une méthode de Newton-Raphson.

**10.** Procédé de traitement d'informations à exécuter dans un dispositif de traitement d'informations comprenant :

une unité de traitement de données configurée pour calculer un paramètre de régression logistique étant un paramètre d'un modèle de régression logistique indiquant une relation entre une première variable et une seconde variable étant deux types différents de données sécurisées associées à chacun d'une pluralité d'échantillons,

où la première variable est une variable explicative et la seconde variable est une variable de résultat, où le dispositif de traitement d'informations est un dispositif de retenue de la variable explicative, le procédé de traitement d'informations comprenant les étapes suivantes :

calculer, par l'unité de traitement de données, un produit interne (t_s) de la première variable et de la seconde variable, l'application d'un calcul sécurisé étant un traitement de calcul appliqué avec des données converties de chacune des variables ; et

calculer le paramètre de régression logistique avec l'exécution d'un traitement de calcul excluant le traitement de calcul du produit interne, en tant que traitement de calcul sans les données converties, où l'unité de traitement de données reçoit un résultat calculé appliqué avec la variable de résultat d'un dispositif de retenue de variable de résultat, et calcule le paramètre de régression logistique avec le résultat calculé appliqué avec la variable de résultat reçue, où le résultat calculé appliqué avec la variable de résultat est une somme totale (t_0) de la variable de résultat.

**11.** Procédé de traitement d'informations à exécuter dans un système de traitement d'informations comprenant :

un dispositif de retenue de variable explicative retenant une variable explicative étant des données sécurisées associées à chacun d'une pluralité d'échantillons ;

un dispositif de retenue de variable de résultat retenant une variable de résultat étant des données sécurisées associées à chacun de la pluralité d'échantillons, le procédé de traitement d'informations comprenant les étapes suivantes :

calculer et délivrer en sortie, par le dispositif de retenue de variable de résultat, une somme totale ($t\_0$) de la variable de résultat associée à chacun de la pluralité d'échantillons au dispositif de retenue de variable explicative ; et

par une unité de traitement de données incluse dans le dispositif de retenue de variable explicative, configurée pour calculer un paramètre de régression logistique étant un paramètre d'un modèle de régression logistique indiquant une relation avec la variable de résultat,

calculer un produit interne ($t\_s$) de la variable explicative et de la variable de résultat avec l'application d'un calcul sécurisé étant un traitement de calcul appliqué avec des données converties de chacune des variables, et calculer le paramètre de régression logistique avec l'application du produit interne ($t\_s$) calculé et de la somme totale ($t\_0$) de la variable de résultat entrée depuis le dispositif de retenue de variable de résultat.

12. Programme destiné à provoquer l'exécution d'un traitement d'informations dans un dispositif de traitement d'informations comprenant une unité de traitement de données configurée pour calculer un paramètre de régression logistique étant un paramètre d'un modèle de régression logistique indiquant une relation entre une première variable et une seconde variable étant deux types différents de données sécurisées associées à chacun d'une pluralité d'échantillons, où la première variable est une variable explicative et la seconde variable est une variable de résultat, où le dispositif de traitement d'informations est un dispositif de retenue de la variable explicative, le programme amenant l'unité de traitement de données à exécuter :

un traitement de calcul d'un produit interne ($t\_s$) d'une première variable et d'une seconde variable, l'application d'un calcul sécurisé étant un traitement de calcul appliqué avec des données converties de chacune des variables ; et

un traitement de calcul du paramètre de régression logistique avec l'exécution d'un traitement de calcul excluant le traitement de calcul du produit interne, en tant que traitement de calcul sans les données converties, où l'unité de traitement de données reçoit un résultat calculé appliqué avec la variable de résultat d'un dispositif de retenue de variable de résultat, et calcule le paramètre de régression logistique avec le résultat calculé appliqué avec la variable de résultat reçue, où le résultat calculé appliqué avec la variable de résultat est une somme totale ($t\_0$) de la variable de résultat.

## FIG. 1

| SAMPLE ID (i) | [OUTCOME VARIABLE (y)] | [EXPLANATORY VARIABLE (x)] | | |
| --- | --- | --- | --- | --- |
| | y1 ONSET OR NON-ONSET OF DISEASE | x1 GENDER (MALE 1, FEMALE 2) | x2 AGE | x3 CHOLESTEROL LEVEL |
| 1 | 0 | 1 | 54 | 213 |
| 2 | 0 | 1 | 36 | 241 |
| 3 | 1 | 2 | 64 | 168 |
| 4 | 1 | 1 | 54 | 213 |
| 5 | 0 | 2 | 40 | 193 |

EP 3 401 828 B1

## FIG. 2

110

INFORMATION
PROCESSING DEVICE A

$\left(\begin{array}{c} \text{OUTCOME-VARIABLE} \\ \text{RETAINING DEVICE} \end{array}\right)$

120

INFORMATION
PROCESSING DEVICE B

$\left(\begin{array}{c} \text{EXPLANATORY-VARIABLE} \\ \text{RETAINING DEVICE} \end{array}\right)$

FIG. 3

# FIG. 4

(A) LEARNING DATA

INFORMATION PROCESSING DEVICE A

INFORMATION PROCESSING DEVICE B

| SAMPLE ID (i) | $\begin{bmatrix} \text{OUTCOME} \\ \text{VARIABLE (y)} \end{bmatrix}$ | [EXPLANATORY VARIABLE (x)] | | |
|---|---|---|---|---|
| | y1 ONSET OR NON-ONSET OF DISEASE | x1 GENDER (MALE 1, FEMALE 2) | x2 AGE | x3 CHOLESTEROL LEVEL |
| 1 | 0 | 1 | 54 | 213 |
| 2 | 0 | 1 | 36 | 241 |
| 3 | 1 | 2 | 64 | 168 |
| 4 | 1 | 1 | 54 | 213 |
| 5 | 0 | 2 | 40 | 193 |

MODELING →

(B) LOGISTIC REGRESSION MODEL

EXPLANATORY VARIABLE: x

$$\text{logit } p(x) = \beta_0 + \beta_1 x_1 + \beta_2 x_2 + \cdots + \beta_r x_r$$

ESTIMATION PARAMETER: $\beta$

NOTE THAT,

$$\text{logit } p(x) = \log\left(\frac{p(x)}{1 - p(x)}\right)$$

EP 3 401 828 B1

# FIG. 5

## (1) SAMPLE UNIT DATA

| SAMPLE ID (i) | [OUTCOME VARIABLE (y)] y1 ONSET OR NON-ONSET OF DISEASE | [EXPLANATORY VARIABLE (x)] x1 GENDER (MALE 1, FEMALE 2) | x2 AGE | x3 CHOLESTEROL LEVEL | PROFILE (No. j) |
|---|---|---|---|---|---|
| 1 | 0 | 1 | 54 | 213 | 1 |
| 2 | 0 | 1 | 36 | 241 | 2 |
| 3 | 1 | 2 | 64 | 168 | 3 |
| 4 | 1 | 1 | 54 | 213 | 1 |
| 5 | 0 | 2 | 40 | 193 | 4 |

## (2) PROFILE UNIT DATA

| (a) PROFILE (No. j) | (b) NUMBER OF SAMPLES ($n_j$) | (c) NUMBER OF SAMPLES ($d_j$) SATISFYING OUTCOME VARIABLE (y)=1 | (d) EXPLANATORY VARIABLE ($x_j$) |
|---|---|---|---|
| 1 | 2 | 1 | (1, 54, 213) |
| 2 | 1 | 0 | (1, 36, 241) |
| 3 | 1 | 1 | (2, 64, 168) |
| 4 | 1 | 0 | (2, 40, 193) |

EP 3 401 828 B1

# FIG. 6

(a) EXEMPLARY PROCESSING OF CALCULATING ADDED RESULT OF SECURE DATA WITH SECURE COMPUTATION

$$(X = x_1 + x_2) \quad (Y = y_1 + y_2)$$

**210**
DEVICE A
$(x_2), (y_2)$

$(x_2), (y_2)$

**S20**

**220**
DEVICE B
$(x_1), (y_1)$

$(x_1), (y_1)$

**S21a**
SECURE-COMPUTATION EXECUTION UNIT OF DEVICE A
$(x_2) + (y_2)$

**S21b**
SECURE-COMPUTATION EXECUTION UNIT OF DEVICE B
$(x_1) + (y_1)$

**S22a**
SECURE-COMPUTATION EXECUTION UNIT OF DEVICE A
$((x_1) + (y_1) + (x_2) + (y_2)) \bmod m = X + Y$

**S22b**
SECURE-COMPUTATION EXECUTION UNIT OF DEVICE A
$((x_1) + (y_1) + (x_2) + (y_2)) \bmod m = X + Y$

EP 3 401 828 B1

## FIG. 7

(b) EXEMPLARY PROCESSING OF CALCULATING MULTIPLIED RESULT OF SECURE DATA WITH SECURE COMPUTATION

$$(X=x_1+x_2) \quad (Y=y_1+y_2)$$

**210**

DEVICE A

$(x_2), (y_2)$

S30

**220**

DEVICE B

$(x_1), (y_1)$

**S31a**
SECURE-COMPUTATION
EXECUTION UNIT OF DEVICE A
INPUT: $(x_2)$
OUTPUT: $(x_2) \times (y_1) + r$

**S32a**
SECURE-COMPUTATION
EXECUTION UNIT OF DEVICE A
INPUT: $(y_2)$
OUTPUT: $(x_1) \times (y_2) + r'$

**S33a**
SECURE-COMPUTATION
EXECUTION UNIT OF DEVICE A
$(x_2) \times (y_2) + (x_2) \times (y_1)$
$+ (x_1) \times (y_2) + r + r'$

1-out-of-m OT

1-out-of-m OT

**S31b**
SECURE-COMPUTATION
EXECUTION UNIT OF DEVICE B
INPUT: $i \times (y_1) + r$
$(i = 0,...,m-1)$

**S32b**
SECURE-COMPUTATION
EXECUTION UNIT OF DEVICE B
INPUT: $i \times (x_1) + r'$
$(i = 0,...,m-1)$

**S33b**
SECURE-COMPUTATION
EXECUTION UNIT OF DEVICE B
$(x_1) \times (y_1) - r - r'$

## FIG. 8

EP 3 401 828 B1

(EXPRESSION a) $\boldsymbol{\beta}^{(k+1)} = \boldsymbol{\beta}^{(k)} + I^{-1}(\boldsymbol{\beta}^{(k)})S(\boldsymbol{\beta}^{(k)})$

ONE-VARIABLE DEPENDENT DATA DEPENDING ON EXPLANATORY VARIABLE (x)

TWO-VARIABLE DEPENDENT DATA DEPENDING ON EXPLANATORY VARIABLE (x) AND OUTCOME VARIABLE (y)

(EXPRESSION b) $\Sigma(\boldsymbol{\beta}) = I^{-1}(\boldsymbol{\beta}) = (X^t V X)^{-1}$

(EXPRESSION b2)

$$X = \begin{bmatrix} 1 & x_{11} & \cdots & x_{1r} \\ 1 & x_{21} & \cdots & x_{2r} \\ \vdots & & \vdots & \\ 1 & x_{J1} & \cdots & x_{Jr} \end{bmatrix}$$

$$V = \begin{bmatrix} n_1 \hat{p}(\boldsymbol{x}_1)(1 - \hat{p}(\boldsymbol{x}_1)) & 0 & \cdots & 0 \\ 0 & n_2 \hat{p}(\boldsymbol{x}_2)(1 - \hat{p}(\boldsymbol{x}_2)) & \vdots & \vdots \\ \vdots & 0 & \vdots & \vdots \\ \vdots & \vdots & \vdots & 0 \\ 0 & 0 & \cdots & n_J \hat{p}(\boldsymbol{x}_J)(1 - \hat{p}(\boldsymbol{x}_J)) \end{bmatrix}$$

(EXPRESSION c) $S(\boldsymbol{\beta}) = \left( \dfrac{dL}{d\beta_0}, \dfrac{dL}{d\beta_1}, \cdots, \dfrac{dL}{d\beta_r} \right)$

$x_{11}$ TO $x_{jr}$: EXPLANATORY VARIABLE

$x_1$ TO $x_j$: EXPLANATORY VARIABLE

(EXPRESSION d)

$$\frac{dL}{d\beta_0} = \sum_{j=1}^{J} (d_j - n_j p(\boldsymbol{x}_j))$$

(EXPRESSION e)

$$\frac{dL}{d\beta_s} = \sum_{j=1}^{J} x_{js} (d_j - n_j p(\boldsymbol{x}_j))$$
$$(s = 1, \ldots, r)$$

$d_j$: NUMBER OF SAMPLES SATISFYING OUTCOME VARIABLE y = 1

$x_j$: EXPLANATORY VARIABLE

$d_j$: NUMBER OF SAMPLES SATISFYING OUTCOME VARIABLE y = 1

$x_{js}$, $x_j$: EXPLANATORY VARIABLE

## FIG. 9

INFORMATION PROCESSING DEVICE A
(OUTCOME-VARIABLE RETAINING DEVICE) — 110

PARAMETER-CALCULATION
EXECUTION UNIT — 111

INPUT UNIT — 131

INNER-PRODUCT
COMPUTATION UNIT — 132

ITERATIVE-COMPUTATION
INPUT-VALUE
GENERATION UNIT — 133

DATA TRANSMISSION/
RECEPTION UNIT — 134

INFORMATION PROCESSING DEVICE B
(EXPLANATORY-VARIABLE RETAINING DEVICE) — 120

PARAMETER-CALCULATION
EXECUTION UNIT — 121

INPUT UNIT — 141

INNER-PRODUCT
COMPUTATION UNIT — 142

DATA TRANSMISSION/
RECEPTION UNIT — 143

ITERATIVE
COMPUTATION UNIT — 144

OUTPUT UNIT — 145

EP 3 401 828 B1

## FIG. 10

INFORMATION PROCESSING DEVICE A
(OUTCOME-VARIABLE RETAINING DEVICE)

START

S101a
INPUT DATA
(OUTCOME VARIABLE: y_i)
$(1 \leq i \leq n)$

S102
CALCULATE INNER PRODUCT (t_s) OF
EXPLANATORY VARIABLE AND OUTCOME
VARIABLE WITH SECURE COMPUTATION

$$t_s = \sum_{i=1}^{n} x_s^i y_i \qquad (1 \leq s \leq r)$$

S103
CALCULATE AND TRANSMIT
SUM TOTAL t_0 OF OUTCOME
VARIABLE (y_i)

$$t_0 = \sum_{i=1}^{n} y_i$$

INFORMATION PROCESSING DEVICE B
(EXPLANATORY-VARIABLE RETAINING DEVICE)

START

S101b
INPUT DATA
(EXPLANATORY VARIABLE: x^i_s)
$(1 \leq i \leq n,\ 1 \leq s \leq r)$

S104
CALCULATE PARAMETERS β_0 TO β_r
WITH ITERATIVE COMPUTATION
OF Newton-Raphson METHOD

S105
OUTPUT PARAMETERS
β_0 TO β_r

END

# FIG. 11

(EXPRESSION a) $\boldsymbol{\beta}^{(k+1)} = \boldsymbol{\beta}^{(k)} + I^{-1}(\boldsymbol{\beta}^{(k)})S(\boldsymbol{\beta}^{(k)})$

ONE-VARIABLE DEPENDENT DATA DEPENDING ON EXPLANATORY VARIABLE (x)

TWO-VARIABLE DEPENDENT DATA DEPENDING ON EXPLANATORY VARIABLE (x) AND OUTCOME VARIABLE (y)

(EXPRESSION b) $\Sigma(\boldsymbol{\beta}) = I^{-1}(\boldsymbol{\beta}) = (X^t V X)^{-1}$

(EXPRESSION b2)
$$X = \begin{bmatrix} 1 & x_{11} & \cdots & x_{1r} \\ 1 & x_{21} & \cdots & x_{2r} \\ \vdots & \vdots & & \vdots \\ 1 & x_{J1} & \cdots & x_{Jr} \end{bmatrix}$$

$$V = \begin{bmatrix} n_1\hat{p}(x_1)(1-\hat{p}(x_1)) & 0 & \cdots & 0 \\ 0 & n_2\hat{p}(x_2)(1-\hat{p}(x_2)) & \vdots & \vdots \\ \vdots & 0 & \vdots & \vdots \\ \vdots & \vdots & \vdots & 0 \\ 0 & 0 & \cdots & n_J\hat{p}(x_J)(1-\hat{p}(x_J)) \end{bmatrix}$$

(EXPRESSION c)
$$S(\boldsymbol{\beta}) = \left( \frac{dL}{d\beta_0}, \frac{dL}{d\beta_1}, \ldots, \frac{dL}{d\beta_r} \right)$$

$x_{11}$ TO $x_{jr}$: EXPLANATORY VARIABLE

$x_1$ TO $x_j$: EXPLANATORY VARIABLE

(EXPRESSION d)
$$\frac{dL}{d\beta_0} = \sum_{j=1}^{J}(d_j - n_j p(x_j))$$

302

$d_j$: NUMBER OF SAMPLES SATISFYING OUTCOME VARIABLE $y = 1$

$x_j$: EXPLANATORY VARIABLE

(EXPRESSION e)
$$\frac{dL}{d\beta_s} = \sum_{j=1}^{J} x_{js}(d_j - n_j p(x_j))$$
$$(s = 1, \ldots, r)$$

301

$d_j$: NUMBER OF SAMPLES SATISFYING OUTCOME VARIABLE $y = 1$

$x_{js}$, $x_j$: EXPLANATORY VARIABLE

# FIG. 12

EP 3 401 828 B1

**INFORMATION PROCESSING DEVICE A**
**(OUTCOME-VARIABLE RETAINING DEVICE)**

**INFORMATION PROCESSING DEVICE B**
**(EXPLANATORY-VARIABLE RETAINING DEVICE)**

(1) PROCESSING FLOW TO BE PERFORMED WITH SECURE COMPUTATION HAVING CONVERTED DATA OF ALL OF EXPLANATORY VARIABLE (x) AND OUTCOME VARIABLE (y) TO BE APPLIED TO ITERATIVE COMPUTATION OF Newton-Raphason METHOD

**START**

**START**

S201a

INPUT DATA
(OUTCOME VARIABLE: $y\_i$)
($1 \leq i \leq n$)

S201b

INPUT DATA
(EXPLANATORY VARIABLE: $x^i\_s$)
($1 \leq i \leq n$, $1 \leq s \leq r$)

S202a

GENERATE CONVERTED DATA
OF OUTCOME VARIABLE: $y\_i$

S202b

GENERATE CONVERTED DATA
OF EXPLANATORY VARIABLE: $x\_i$

S203

PERFORM ITERATIVE COMPUTATION OF Newton-Raphason METHOD
PERFORM SECURE COMPUTATION APPLIED WITH
CONVERTED DATA OF ALL OF FOLLOWING PIECES OF DATA

(1) EXPLANATORY VARIABLES BEING CONSTITUENT ELEMENTS OF MATRIX X AND MATRIX Y EXPRESSED IN (EXPRESSION b2) OF FIG. 8

(2) EXPLANATORY VARIABLES AND OUTCOME VARIABLES INCLUDED IN (EXPRESSION d) OF FIG. 8

(3) EXPLANATORY VARIABLES AND OUTCOME VARIABLES INCLUDED IN (EXPRESSION e) OF FIG. 8

S204

OUTPUT PARAMETERS $\beta\_0$ TO $\beta\_r$

**END**

# FIG. 13

**INFORMATION PROCESSING DEVICE A
(OUTCOME-VARIABLE RETAINING DEVICE)**

(2) PROCESSING FLOW ACCORDING TO PRESENT DISCLOSURE
TO BE PERFORMED WITH SECURE COMPUTATION ONLY
FOR CALCULATION PROCESSING OF INNER PRODUCT (t_s)
OF EXPLANATORY VARIABLE (x) AND OUTCOME VARIABLE (y)

**INFORMATION PROCESSING DEVICE B
(EXPLANATORY-VARIABLE RETAINING DEVICE)**

START

START

S301a
INPUT DATA
(OUTCOME VARIABLE: y_i)
$(1 \leq i \leq n)$

S301b
INPUT DATA
(EXPLANATORY VARIABLE: $x^i\_s$)
$(1 \leq i \leq n,\ 1 \leq s \leq r)$

S302a
GENERATE CONVERTED DATA
OF OUTCOME VARIABLE: y_i

S302b
GENERATE CONVERTED DATA
OF EXPLANATORY VARIABLE: x_i

S303
CALCULATE INNER PRODUCT (t_s) OF EXPLANATORY
VARIABLE AND OUTCOME VARIABLE WITH
SECURE COMPUTATION
$$t_s = \sum_{i=1}^{n} x_s^i y_i$$

S304
CALCULATE AND TRANSMIT SUM
TOTAL t_0 OF OUTCOME VARIABLE (y_i)
$$t_0 = \sum_{i=1}^{n} y_i$$

S305
CALCULATE PARAMETERS $\beta\_0$ TO $\beta\_r$
WITH ITERATIVE COMPUTATION OF
Newton-Raphson METHOD

S306
OUTPUT PARAMETERS $\beta\_0$ TO $\beta\_r$

END

# FIG. 14

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011083101 A

- JP 2009199068 A

**Non-patent literature cited in the description**

- **O. GOLDREICH ; S. MICALI ; A. WIGDERSON.** *How to play any mental game. STOC'87,* 1987, 218-229